(19) Europäisches Patentamt
European Patent Office
Office européen des brevets

(11) Publication number: **0 688 601 A1**

(12) # EUROPEAN PATENT APPLICATION

(21) Application number: **95201082.5**

(51) Int. Cl.[6]: **B01F 17/00**

(22) Date of filing: **29.06.90**

This application was filed on 27 - 04 - 1995 as a divisional application to the application mentioned under INID code 60.

(30) Priority: **08.08.89 US 391187**
**27.06.90 US 542780**

(43) Date of publication of application:
**27.12.95 Bulletin 95/52**

(60) Publication number of the earlier application in accordance with Art.76 EPC: **0 486 510**

(84) Designated Contracting States:
**BE CH DE DK ES FR GB IT LI NL SE**

(71) Applicant: **STEPAN COMPANY**
**22 West Frontage Road**
**Northfield,**
**Illinois 60093 (US)**

(72) Inventor: **Goze, Jean M.**
**757 N.E. Holcomb Drive**
**Mundelein,**
**IL 60060 (US)**
Inventor: **Bernhardt, Randal J.**
**1905 E. Fairfield Road**
**Lindenhurst,**
**IL 60046 (US)**
Inventor: **Hartlage, James A.**
**3926 Britanny Drive**
**Northbrook,**
**IL 60062 (US)**
Inventor: **Smith, Norman R.**
**90 East Atteridge Road**
**Lake Forest,**
**IL 60045 (US)**
Inventor: **Lietz, Dennis E.**
**935 Knollwood Road**
**Deerfield,**
**IL 60015 (US)**
Inventor: **Kay, James W.**
**Minehill Road**
**Durham,**
**PA 18039 (US)**
Inventor: **Breitbeil, Fred W.**
**831 S. Dryden Place**
**Arlington Heights,**
**IL 60005 (US)**
Inventor: **Sajic, Branco**
**5048 Ridgeway Ave.**
**Chicago,**
**IL 60625 (US)**
Inventor: **Rockwell, Ned Miles**
**301 Neuman Ct.**
**Lake Bluff,**
**IL 60044 (US)**
Inventor: **McConnell, Nina**
**Montserrat,**
**894 Burr Avenue**
**Winnetka,**
**IL 60093 (US)**
Inventor: **Mohring, William Richard**
**4400 Lake Avenue**
**Glenview,**
**IL 60025 (US)**

(74) Representative: **Guerre, Dominique**
**CABINET GERMAIN et MAUREAU**
**Le Britannia Tour C**
**20 Bd E. Deruelle**
**F-69392 Lyon Cédex 03 (FR)**

(54) **Cyclic amidocarboxy surfactants, synthesis and use thereof**

(57) A composition comprising an emulsion of an oil phase and a water phase, wherein the emulsion comprises an emulsifier of the formula:

EP 0 688 601 A1

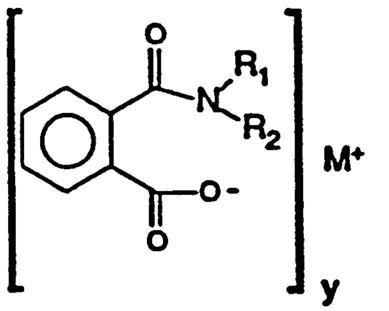

(I)

wherein:
R represents $C_1$-$C_{40}$ alkyl, $C_1$-$C_{40}$ alkenyl, alkylaryl where the alkyl portion is $C_1$-$C_{40}$ alkyl, aryl, $C_3$-$C_7$ cycloalkyl, or $R_1$-O-$R_2$ where $R_1$ and $R_2$ independently represent $C_1$-$C_{22}$ alkyl, $C_1$-$C_{22}$ alkenyl, alkylaryl where the alkyl portion is $C_1$-$C_{22}$ alkyl, aryl, or $C_3$-$C_7$ cycloalkyl;
x represents a cation;
m is an integer satisfying the valency of X; and
the composition is at a pH of 5.0 to 10.

## Field of the Invention

The invention relates to surfactants and somewhat more particularly, to improved amidocarboxy compounds, compositions containing such compounds, methods of preparing such compounds and compositions, as well as methods of using such compounds or compositions in a wide variety of technically useful fields.

## Background of the Invention

A wide variety of surface active (surfactant) compounds are known and widely used. Further, certain relatively specific phthalamate derivatives are at least nominally disclosed in academic and patent literature. Some specific phthalamate derivatives have been suggested as being useful in plant growth regulator formulations, insect repellent formulations, bactericidal, fungicidal, herbicidal formulations, additives for improving low temperature flow characteristics of petroleum distillate fuels, solvent extraction formulations for certain heavy metal ions, catalyst systems for polyurethane foam formulations, additives for thermal recording materials, thickeners for silicone grease and oil-based drilling muds, additives for water-insensitive coatings, plasticizers, etc. For example, U.S. Patent 2,101,323 provides a scant disclosure of certain monoamides of particular dicarboxylic acids including cyclic amidocarboxy compounds and various phthalamate derivatives, solvent-based synthesis routes and a recitation of potential uses therefore. Somewhat similarly, U.S. Patent 2,467,192 provides a disclosure of certain ammonium salts of "amic" acids, including various phthalamate derivatives, synthesis thereof and a use therefore as a plasticizer which may involve forming water-insensitive surfaces or films. Numerous other, relatively remote, disclosures involving phthalamic acids or phthalamate derivatives include the following U.S. Patents: 2,554,249 (additive for insecticidal compositions); 2,582,670 (additive for vulcanization activators); 2,742,496 (additive for rust and corrosion inhibitor formulations); 3,095,286 (additive to screen-clogging prevention and rust inhibition formulations); 4,211,534 (additive for improving low temperative flow characteristics of petroleum fuel oils); 4,478,958 (additive to catalyst systems for polyurethane foam formulations); etc.

In addition, so-called fast-breaking personal-care compositions involving vinyl polymers are disclosed in European Patent Application Number 02 68 164 A2. Further, an oil-in-water hair/skin conditioning composition containing certain water-insoluble, unctuous oleaginous materials and a water-dispersable hydrated polyvalent metal sail (i.e. Al), adjusted to a relatively low pH so as to invert to a water-in-oil emulsion when rubbed onto hair/skin and provide a moisturizing or protective barrier is disclosed in U.S. Patent 4,551,330.

Further, U.S. Patents 3,928,432 and 4,544,726 disclose certain monomeric latex polymerization surfactants, which during polymerization are chemically incorporated into the polymers so as to prevent migration thereof and thereby reduce water-sensitivity of the resulting polymeric mass.

U.S. Patent 4,741,855 describes shampoo compositions which comprise a synthetic surfactant, an insoluble, non-volatile silicone, a suspending agent, and water. The described suspending agents include long chain esters of ethylene glycol, esters of long chain fatty amine oxides and many others. There appear to be several key conditioning components in these compositions, including an insoluble, non-volatile, silicone, a suspending agent and a quaternary ammonium compound. The quaternary ammonium compounds, disclosed in U.S. Patent No. 4,741,855 as ingredients in a shampoo composition are di-(hydrogenated tallow) dimethyl ammonium chloride and cetyltrimethyl ammonium chloride.

The use of silicone material in shampoos has been described in a number of different publications. The manufacture of such compositions is extremely complicated and requires specialized mixing equipment, high shear pumps, a heat exchanger, several manufacturing tanks, etc.

Quaternary ammonium compounds derived from fatty acid amines such as tallow amine and di-tallow amine have been used as conditioners, surfactants and thickeners or emulsifiers in various shampoo and hair care products. For example, European Patent Application No. 0067635A2 discloses conditioning shampoos containing quaternary ammonium compounds of the formula:

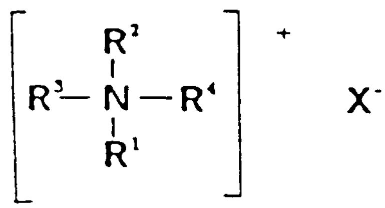

wherein the $R^1$ and $R^2$ groups contain an average of from about 16 to 22 carbon atoms, most preferably from about 16 to about 18 carbon atoms, $R^3$ and $R^4$ are $C_1$ to $C_4$ alkyl or hydroxyalkyl groups, and X is any compatible anion, particularly one selected from the group consisting of halide, hydroxide, methylsulfate, or acetate anions.

The shampoo compositions of that patent application also contain acyl derivatives which are long chain amides, alkanolamides, esters of ethylene glycol and glycerine, esters of carboxylic acids, esters of thiodicarboxylic acids, and mixtures of these derivatives. The shampoo compositions of that patent application also contain surfactants which are represented by the formula:

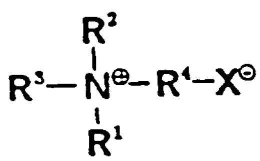

where in $R^1$ is a long chain alkyl radical having from about 10 to about 18 carbon atoms or an amido radical represented by the formula:

$R^5$-CONH$(CH_2)_3$

wherein $R^5$ is a long chain alkyl radical, $R^2$ and $R^3$ are each alkyl radicals having from about 1 to about 3 carbon atoms. $R^4$ is an alkylene or hydroxy alkylene radical having from about 1 to about 4 carbon atoms, and X is a carboxylate radical.

European Patent Application No. 0 152 194 A2 discloses shampoo compositions containing quaternary ammonium salts of the formula:

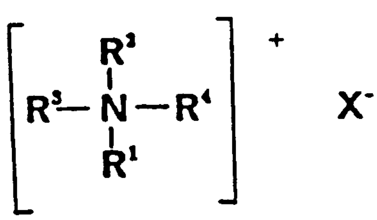

wherein $R_1$ is hydrogen, or an aliphatic group of from 1 to 22 carbon atoms, or an aromatic, aryl or alkaryl group having 6 to 20 carbon atoms; $R_2$ is an aliphatic group having from 12 to 22 carbon atoms; $R_3$ and $R_4$ are each alkyl groups having from 1 to 3 carbon atoms; and X is an anion selected from halogen, acetate, phosphate, nitrate and methyl sulfate radicals.

U.K. Patent Application No. GB 2196979 A provides hair care compositions comprising compounds of the formula:

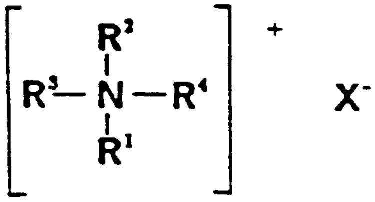

wherein $R_1$ and $R_2$ are aliphatic groups containing from about 12 to about 22 carbon atoms, $R_3$ and $R_4$ are hydrogen or short chain alkyl groups containing from about 1 to about 4 carbon atoms and X is anion selected from halogen, acetate, phosphate, nitrate and alkyl sulfate radicals.

U.K. Patent Application No. GB 2124647 A teaches quaternary ammonium compounds useful in shampoo compositions. The ammonium compounds have the formula:

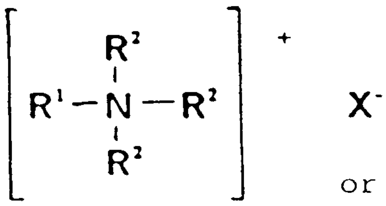

or

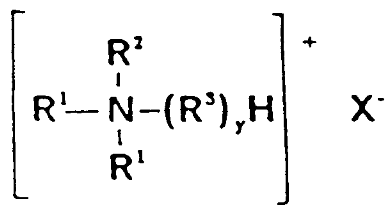

wherein $R_1$ is an aliphatic alkyl group containing an average of from about 16 to 22 carbon atoms, most preferably from about 16 to about 18 carbon atoms, the $R_2$ groups are $C_1$ to $C_4$ alkyl or hydroxylalkyl groups, the $R_3$ groups are alkylene oxide groups, preferably propylene oxide, where y is 1-4 and X is any compatible anion, particularly one selected from the group consisting of halide, hydroxide, methylsulfate, or acetate anions.

European Patent Application No. 0294 894 discloses conditioning agents for delivery from shampoos comprising compounds of the formula:

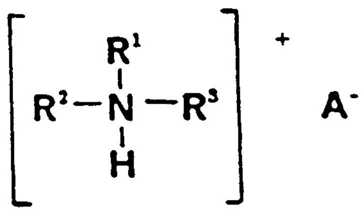

wherein $R_1$ and $R_2$ can independently be $C_{16}$ to $C_{20}$ alkyl or alkenyl and $R^3$ is H or $CH_3$, and A is an anionic surfactant selected from the group consisting of alkyl sulfonates, aryl sulfonates, alkylaryl sulfonates, alkyl sulfates, alkyl ethoxylated sulfates, dialkyl sulfosuccinates, ethoxylated alkyl sulfonates, alkyl oxybenzene sulfonates, acyl isethionates, acyl alkyl taurates, olefin sulfonates and paraffin sulfonates.

A wide variety of surface active (surfactant) compounds are known and widely used. Further, certain relatively specific phthalamate derivatives are at least nominally disclosed in academic and patent literature. Some specific phthalamate derivatives have been suggested as being useful in plant growth regulator formulations, insect repellent formulations, bactericidal, fungicidal, herbicidal formulations, additives for improving low temperature flow characteristics of petroleum distillate fuels, solvent extraction formulations for certain heavy metal ions, catalyst systems for polyurethane foam formulations, additives for thermal recording materials, thickeners for silicone grease and oil-based drilling muds, additives for water-insensitive coatings, plasticizers, etc. Phthalamic acids or phthalamate derivatives have been used as additives for insecticidal compositions; additives for vulcanization activators; additives for rust and corrosion inhibitor formulations; additives to screen-clogging prevention and rust inhibition formulations; additives for improving low temperative flow characteristics of petroleum fuel oils; additives to catalyst systems for polyurethane

foam formulations.

Ammonium phthalamates have been used as additives in fuel oil compositions, blending agents for grease, lubricating oil additives, and thickening agents for lubricating oil compositions. These ammonium phthalamates have the formula

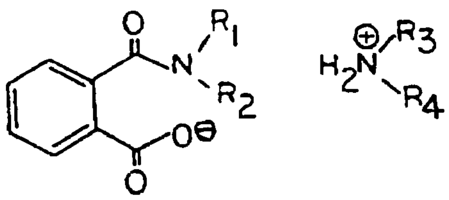

wherein

$R_1$, $R_2$, $R_3$ and $R_4$ are the $C_{16}$-$C_{40}$, preferably $C_{16}$-$C_{24}$ straight chain alkyl groups of secondary amine, and may be the same or different.

Tallow is a fatty acid byproduct of the meat-packing industry obtained by rendering the body fat from cattle and sheep. Tallows from different sources vary in free fatty acid content. The fatty acids normally found in callow are myristic acid, palmitic acid, stearic acid, oleic acid, and linoleic acid.

Several methods are known for the preparation of tallow amines, but the most common method in industry is the conversion of a fatty acid to a nitrile by treating with ammonia, followed by catalytic hydrogenation of the nitrile to primary, secondary, or tertiary amine by suitable adjustment in the reaction conditions. Tallow amines, as well as di(hydrogenated tallow) amine, are commercially available: for example, di(hydrogenated tallow) amines are available under the trade name ARMEEN 2HT™ (Akzo Chemicals, Chicago, Illinois).

Various routes exist for the preparation of phthalamic acids and phthalamic acid salts. In U.S. Patent No. 4,402,708 N,N-diarachidyl phthalamic acid was prepared by adding phthalic anhydride to a 40% solution of amine in toluene in a 1/1 mole ratio at 80 °C. The product was recovered by vacuum drying at 50 °C, 0.05 mmHg for 20.5 hours. Phthalic anhydride sublimation was observed. This method makes no mention of the presence of any ammonium salt in the resultant product.

U.S. Patent No. 4,402,708 describes a method for preparing N,N-dioctadecyl phthalamic acid dioctadecyl ammonium salt and N,N-diarachidyl phthalamic acid diarachidyl ammonium salt. Phthalic anhydride was added to a 10% solution of amine in toluene in an anhydride to amine mole ratio of 1/2. The product was recovered by filtering and film evaporating a 1/1 toluene/n-heptane solution at 55 °C, 40 mmHg.

Phthalamic acids have also been prepared by melting phthalic anhydride at 131 °C and subsequent addition of molten secondary amine. The reactants to be added in an equimolar ratio. At the temperature used in this method, 131 °C, excessive phthalic anhydride sublimation occurs and increased product degradation is observed. This method makes no mention of the presence of any ammonium salt in the resultant product.

Phthalamic acids have been prepared by addition of a solution of secondary amine in isopropanol at 78C to a phthalic anhydride/isopropanol slurry at 78 °C in a one to one phthalic anhydride/amine molar ratio with subsequent vacuum stripping of the solvent. This method utilizes isopropanol as the solvent for the reaction. Isopropanol, a secondary amine, reacts with phthalic anhydride to yield isopropyl mono ester of phthalic acid. At 78 °C, as much as 40% of the product may be this ester.

Each of these methods produces a mixture of the desired phthalamic acid and an ammonium phthalamate salt. However, these methods make no mention of the presence of any ammonium salt in the resultant product.

However, while various amidocarboxy compounds, including certain fatty phthalamates may be suggested in certain arts, and some fast-breaking emulsions may be suggested for personal-care products as well as some latex polymerization surfactants capable of providing water-insensitive films or coatings may be known, the surfactant arts have not heretofore recognized the specific materials, synthesis routes and uses disclosed herein and which exhibit superior surfactant/emulsification characteristics useful in a wide variety of technical applications including surfactants per se, caustic-stabile surfactants, chlorine-stabile surfactants, fast-break emulsifiers for personal-care products, emulsifiers for agricultural chemicals, domestic fabric softeners, destructible latex polymerization surfactants, domestic detergent additives, emulsifiers for portland cement and concrete, floatation/benefication additives for various mineral ores, additives for electroplating and/or surface finishing baths for metal goods, additives for plaster, gypsum end miscellaneous building materials, additives for enhanced oil recovery formulations, wetting and lubricating surfactants for textile processing, pulp digestive additives, surfactants for polyurethane/ isocyanurate foam formulation, pour-point depressant for transporting viscous petroleum oils, industrial surfactants for emulsifying a wide variety of oily materials, as suspending agents for various particular materials, etc.

The invention provides surfactants of a general formula:

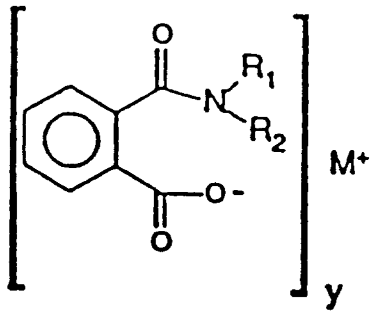

(I)

wherein $R_1$ and $R_2$ we independently selected from the group consisting essentially of H or $C_1$ - $C_{40}$ linear or branched, substituted or unsubstituted alkyl, cycloalkyl, alkylene, alkaryl, aryl or $R_3$-O-$R_4$ groups, and mixtures thereof with $R_3$ and $R_4$ being independently selected from the group consisting essentially of $C_1$ - $C_{22}$ linear or branched, substituted or unsubstituted alkyl, cycloalkyl, alkylene, alkaryl, and aryl groups; y is an integer of a value satisfying the valency of M; and M is a cation; synthesis routes for producing such surfactant at relatively high yields with little or no purification and methods of utilizing such surfactants

The present invention also provides surfactant solutions comprising an effective amount of a salt of the following general:

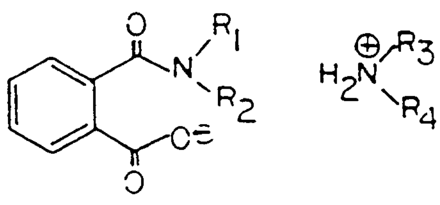

(II)

wherein

$R_1$, $R_2$, $R_3$, and $R_4$ are the same or different and represent straight or branched chain alkyl groups having 10 to 40 carbon atoms, or aryl straight or branched chain alkyl groups having 10 to 40 carbon atoms.

The present invention also provides surfactant solutions comprising an effective surfactant amount of an acid of the following general formula:

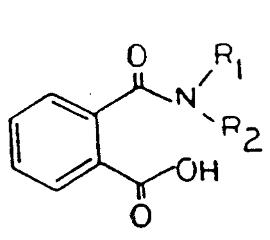

(III)

wherein

$R_1$ and $R_2$ are the same or different and represent straight or branched chain alkyl groups having 10 to 40 carbon atoms, or aryl straight or branched chain alkyl groups having 10 to 40 carbon atoms.

The present invention also provides surfactant solutions comprising an effective surfactant amount of a mixture of a salt of formula II above and an acid of formula III.

In addition, the present invention provides formulated conditioning shampoos comprising an effective conditioning amount of a salt of formula II. The invention further provides a formulated conditioning shampoo comprising an effective conditioning amount of a mixture of a salt of formula II above and an acid

salt of formula III above.

The present invention also provides emulsifier solutions comprising an effective emulsifier amount of a salt of formula I above. The present invention also provides emulsifier solutions comprising an effective emulsifier amount of a mixture of a salt of formula I above and an acid of formula II above.

These surfactants are useful as surfactants per se, as caustic-stabile surfactants, as chlorine-stable surfactants, as cosmetic emulsifiers for personal care product, such as skin creams, skin lotions, hair conditioners, etc., as fast-breaking skin-care product emulsifiers; as emulsifiers for agricultural chemicals, as domestic fabric softeners; as destructible latex polymerization surfactants in coating and/or adhesive systems, as domestic detergent additives, such as in heavy-duty detergents, in light-duty detergents, in dishwashing detergents, in various hard surface cleaners, etc.; as emulsifiers for portland cement and concrete; as floatation/benefication additives for various mineral ores; as additives for electroplating and/or surface finishing baths for metal goods; as additives for plaster, gypsum and miscellaneous building materials; as enhanced oil recovery additives; as wetting and lubricating surfactants for textile processing; as pulp digestive additives; as surfactants for polyurethane/isocynaurate foam systems; as pour-point depressants for transporting viscous petroleum oils; as industrial surfactants for emulsifying a wide variety of oily materials; as low temperature stabilizers for fatty alcohol/water emulsions; as suspending agents for various particulate material, such as coal tar, sulfur or coal; etc.

Accordingly, it is an object of the invention to provide surfactants of the general formula (I) above. In certain embodiments of this feature of the invention, $R_1$ and $R_2$ are independently selected from the group consisting essentially of H or $C_1$ - $C_{30}$ linear or branched, substituted or unsubstituted alkyl, cycloalkyl, alkylene, alkaryl, aryl or $R_3$-O-$R_4$ groups, with $R_3$ and $R_4$ being independently selected for the group consisting essentially of $C_1$ - $C_{22}$ linear or branched, substituted or unsubstituted, alkyl, cycloalkyl, alkylene, alkaryl and aryl groups. In more preferred embodiments, $R_1$ and $R_2$ are independently selected from the group consisting essentially of H or $C_1$ - $C_{22}$ and more preferably are selected from the group consisting of H or $C_1$ - $C_{18}$ alkyl groups. The cation, M, is preferably selected from the group consisting essentially of H, Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives], Ba, Ca, Mg, Al, Ti, Zr and mixtures thereof and even more preferably is selected from the group consisting essentially of H, Na, K, $NH_4$ [including $(CH_3CH_2)_3NH$. $(CH_3CH_2)_2NH_2$, $(HOCH_2CH_2)_3NH$, $(HOCH_2CH_2)_2NH_2$ and similar ammonium derivatives] and mixtures thereof. Specific preferred exemplary embodiments of this feature of the invention include
sodium N-dodecylphthalamate,
sodium N-octadecylphthalamate,
sodium N-hexadecylphthalamate,
sodium N-tallowphthalamate,
sodium N-cocophthalamate,
sodium N-isododecyloxypropylphthalamate,
sodium N-methyl-N-dodecylphthalamate,
sodium N-methyl-N-octadecylphthalamate,
sodium N-methyl-N-hexadecylphthalamate,
sodium N-methyl-N-tallowphthalamate,
sodium N-methyl-N-cocophthalamate,
N, N- di (hydrogenated tallow) phthalamic acid
N N - di (hydrogenated tallow) phthalamic acid di(hydrogenated tallow) ammonium salt
mixtures thereof and their corresponding potassium, ammonium, triethylammonium and triethanolammonium salts. Certain of the surfactants (I) above are caustic-stabile, i.e., function and do not break-down in relatively high pH environments, while certain of the dialkyl surfactants are chlorine-stabile, i.e., function and do not breakdown in environments having a relatively high chlorine-ion concentration.

It is a further object of the invention to provide methods of producing surfactants of formula (I) above. In certain preferred embodiments of this feature of the invention, molten phthalic anhydride may be brought into contact with at least a primary ($R_5NH_2$) amine, wherein $R_5$ is selected from the group consisting essentially of $C_1$ - $C_{40}$ linear or branched, substituted or unsubstituted alkyl, cycloalkyl, alkylene, alkaryl, aryl, or $R_3$-O-$R_4$ groups, with $R_3$ and $R_4$ being independently selected from the group consisting essentially of $C_1$ - $C_{22}$ linear or branched, substituted or unsubstituted alkyl, cycloalkyl, alkylene, alkaryl and aryl groups, under reaction conditions conducive for formation of an imide between the anhydride and the amine, and a nucleophilic source yielding a cation selected from the group consisting essentially of Na, K, $NH_4$ and mixtures thereof may be added to the imide under reaction conditions conducive to formation of the corresponding alkali salts. In certain forms of this embodiment, a hydroxide of an alkaline-earth metal may be added, after addition of the nucleophilic source, to form the corresponding alkaline-earth salt.

Further, in yet other forms of this embodiment, a mineral acid may be added after the formation of the alkali salt and thereafter a $C_1$ - $C_4$ alkoxide of a metal selected from the group consisting essentially of Al, Ti, Zr and mixtures thereof or at least one amine, may be added to form the corresponding salt. In other preferred embodiments of this feature of the invention, molten phthalic anhydride may be brought into contact with at least a secondary ($R_5R_6NH$) amine, wherein $R_5$ and $R_6$ are independently selected from the group consisting essentially of $C_1$ - $C_{40}$ linear or branched, substituted or unsubstituted, alkyl, cycloalkyl, alkylene, alkaryl, aryl, or $R_3$-O-$R_4$ groups with $R_3$ and $R_4$ being independently selected from the group consisting essentially of $C_1$ - $C_{22}$ linear or branched, substituted or unsubstituted alkyl, cycloalkyl, alkylene, alkaryl and aryl groups, under reaction conditions conducive for formation of phthalamic acid between the anhydride and the amine, and a source yielding a cation selected from the group consisting essentially of Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives] Ba, Ca, Mg, Al, Ti, Zr and mixtures thereof may be added to the phthalamic acid so as to form the corresponding salt. In yet other preferred embodiments of this feature of the invention, phthalic anhydride may be brought into contact with a tertiary $C_1$ - $C_{40}$ amine so as to obtain an admixture thereof and a primary or secondary ($R_5R_6NH$) amine, wherein $R_5$ and $R_6$ are independently selected from the group consisting essentially of H or $C_1$ - $C_{40}$ linear or branched, substituted or unsubstituted alkyl, cycloalkyl, alkylene, alkaryl, aryl, or $R_3$-O-$R_4$ groups, with $R_3$ and $R_4$ being independently selected from the group consisting essentially of $C_1$ - $C_{22}$ linear or branched, substituted or unsubstituted alkyl, cycloalkyl, alkylene, alkaryl and aryl groups, may be added to the admixture under reaction conditions conducive for formation of an alkyl phthalamate salt. The primary or secondary ($R_5R_6NH$) amine may be added sequentially to or substantially simultaneously with the tertiary amine.

It is a further object of the invention to provide a foodstuff emulsifier having the general formula (I) above; as well as a foodstuff composition which maybe comprised of about 0.01 percent to about 5.0 percent by weight of the foodstuff emulsifier, and an amount up to about 99.99 percent by weight of a food-grade material, as well as a method of producing prepared foodstuff wherein select unprepared foodstuff ingredients may be admixed with about 0.01 to about 5.0 percent actives of the foodstuff emulsifier having the formula (I) above and subjecting the resulting admixture to preparation conditions sufficient to produce a prepared foodstuff. In certain preferred embodiments of this feature of the invention, the foodstuff emulsifier comprised of formula (I) above, includes specie wherein $R_1$ and $R_2$ are independently selected from the group consisting essentially of H or $C_1$ - $C_{18}$ alkyl groups and mixtures thereof and the cation is sodium or potassium.

It is yet a further object of the invention to provide an emulsifier for skin-care products having the general formula (I) above; a solvent-tolerant skin-care composition which may comprise about 0.01 percent to about 10.0 percent of a primary emulsifier having the general formula (I) above, about 0.01 percent to about 10.0 percent of a secondary emulsifier, and a sufficient amount of an emollient to attain a ratio of emollient to emulsifier in the range of about 3:1 to about 30:1. In certain forms of this embodiment, the skin-care compositions may be relatively salt-tolerant. In yet other embodiments of this feature of this invention, the skin-care composition may exhibit a pH value in the range of about 5 to about 12 In certain preferred forms of the emulsifier for skin-care products having the formula (I) above, $R_1$ and $R_2$ are independently selected from the group consisting essentially of H or $C_1$ - $C_{18}$ alkyl groups and mixtures thereof and M, the cation, may selected from the group consisting essentially of Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives] Ba, Ca, Mg, Al, Ti, Zr and mixtures thereof and more preferably may selected from the group consisting essentially of Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives] and mixtures thereof.

It is yet a further object of the invention to provide a hair-care composition comprising about 0.01 percent to about 10.0 percent of a conditioning surfactant having the general formula (I) above, about 10 percent to about 25 percent of a hair-cleansing material; and Q.S. 100 of an aqueous material. In certain preferred embodiments of this conditioning surfactant, $R_1$ and $R_2$ are independently selected from the group consisting essentially of H or $C_1$ - $C_{18}$ alkyl groups and mixtures thereof. Specific exemplary embodiments of conditioning surfactants for hair-care products or compositions include materials selected from the group consisting essentially of
sodium N-dodecylphthalamate,
sodium N-octadecylphthalamate,
sodium N-hexadecylphthalamate,
sodium N-tallowphthalamate,
sodium N-cocophthalamate, sodium N-isododecyloxypropylphthalamate, mixtures thereof and their corresponding potassium, ammonium, triethylammonium and triethanolammonium salts and mixtures thereof.

A particularly attractive object of the invention comprises applying to human skin a relatively salt-tolerant, relatively solvent-tolerant, and relatively fast-breaking anionic oil-in-water cosmetic composition having a pH in the range of about 5 to 12, with such composition being comprised of about 0.01 percent to about 10.0 percent of a primary, relatively high HLB emulsifier having the general formula (I) above; about 0.01 percent to about 10.0 percent of a secondary, relatively low HLB emulsifier, and a sufficient amount of an emollient to attain a ratio of emollient to emulsifier in the range of about 3:1 to about 30:1 at a select skin area; and rubbing the composition into the skin area for a relatively brief period of time sufficient to break the composition and allow the emollient to penetrate into the skin area. A somewhat similar embodiment of this feature of the invention comprises conditioning or treating hair (to avoid tangles, snarls, etc.) by applying to wet hair a shampoo composition comprised of about 0.05 percent to about 10.0 percent of a water-dispersable anionic surfactant having the general formula (I) above; about 10 percent to about 25 percent of a hair-cleansing material; and Q.S. 100 of aqueous material; admixing such shampoo composition with the wet hair and rinsing the resultant hair with water or aqueous solution so as to substantially remove any free shampoo composition.

It is also an object of the invention to provide an emulsified oil-in-water composition comprising about 0.01 to about 10.0 percent of a relatively high HLB emulsifier having the general formula (I) above; about 0.01 percent to about 10.0 percent of a relatively low HLB emulsifier; up to about 80 percent of an oily material and Q.S. 100 water. The oily material may be preferably selected from a group consisting essentially of crude petroleum oil, distilled petroleum oil, heavy paraffinic oil, asphaltene oil, linseed oil, tall oil, soybean oil alkyd, linseed oil alkyd, mineral oil, petrolatum, isopropyl palmitate, isopropyl myristate, caprylic/capric triglyceride, lanolin, acetylated lanolin alcohol, dimethicone, hydrogenated vegetable oil, sesame oil, safflower oil, avocado oil, glycerine, propylene glycol, sorbitol, $C_{12}$ - $C_{16}$ alcohol benzoates, cyclomethicone, dimethicone, cocoa butter, vitamin E acetate, squalane, sodium pyrolidone carboxylic acid, methyl glucose ether, panthenol, melanin, mixtures thereof, etc.

It is yet another object of the invention to provide an emulsifier having the general formula (I) above, for agricultural chemicals and agricultural chemical compositions comprising, in combination, an amount up to about 10 percent, by weight of the above emulsifier and an effective amount of an active agricultural chemical in a suitable carrier, such as an oil or an aqueous system. A feature of this embodiment of the invention involves applying an active agricultural herbicide/pesticide to a growing crop so as to avoid post-application washoff and comprises producing a sprayable emulsion containing an effective amount of an active agricultural herbicide/pesticide in a suitable carrier and an amount of up to about 10 percent of a destructible surfactant having the general formula (I) above; spraying such emulsions onto the growing crop; and subjecting the sprayed emulsion to ambient temperature conditions over a period of time whereby the surfactant chemically changes so that the resulting agricultural chemical composition becomes substantially water-insensitive. Also, the desired chemical change may be accomplished by subjecting the sprayed crop to a pH environment of less than about 5.

It is yet a further object of the invention to provide a fabric softener having the general formula (I) above, as well as a method of providing softness characteristics to fabrics by adding an amount of such softener to fabrics undergoing cleansing in a domestic washing machine, substantially uniformly dispersing the surfactant throughout such fabrics and drying the fabrics. In certain preferred embodiments of this feature of the invention, the surfactant may be added to the rinse cycle of the machine and in other preferred embodiments of this feature, the surfactant may be added to the wash cycle of domestic washing machines, along with a detergent composition. In certain preferred embodiments of this feature of the invention, $R_1$ and $R_2$ in formulation (I) above, may be selected from the group consisting essentially of H or $C_1$ - $C_{18}$ alkyl groups and mixtures thereof and the cation in the above formulation may be selected from a group consisting essentially of Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives] and mixtures thereof.

A feature of this object of the invention is a fabric softener prepared by contacting molten phthalic anhydride with a least a primary ($R_5NH_2$) amine wherein $R_5$ is selected from the group consisting essentially of $C_1$ - $C_{40}$ linear or branched, substituted or unsubstituted alkyl, cycloalkyl, alkylene, alkaryl, or $R_3$-O-$R_4$ groups, with $R_3$ and $R_4$ being independently selected from a group consisting essentially of $C_1$ - $C_{22}$ linear or branched, substituted or unsubstituted alkyl, cycloalkyl, alkylene, alkaryl, and aryl groups under reaction conditions conducive for formation of an imide between the anhydride and the amine, and adding to the imide, a nucleophilic source yielding a cation selected from the group consisting essentially of Na, K, $NH_4$ and mixtures thereof to form a corresponding alkali salt.

It is yet another object of the invention to provide a "destructible" latex emulsion polymerization surfactant prepared by contacting phthalic anhydride with at least a tertiary $C_1$ - $C_{40}$ amine so as to obtain a mixture of the anhydride and the amine, and adding a primary or a secondary ($R_5R_6NH$) amine to the

admixture, with $R_5$ and $R_6$ being independently selected from the group consisting essentially of H or $C_1$ - $C_{40}$ linear or branched, substituted or unsubstituted alkyl, cycloalkyl, alkylene, alkaryl, aryl, or $R_3$-O-$R_4$ groups, with $R_3$ and $R_4$ being independently selected from a group consisting essentially of, $C_1$ - $C_{22}$ linear or branched, substituted or unsubstituted alkyl, cycloalkyl, alkylene and aryl groups, under reaction conditions conducive for formation of an alkylphthalamate salt. In certain embodiments of this feature of the invention, a latex polymerization surfactant is prepared by contacting molten phthalic anhydride with at least a primary ($R_5NH_2$) amine, wherein $R_5$ is selected from the group consisting essentially of $C_1$ - $C_{40}$ linear or branched, substituted or unsubstituted alkyl, cycloalkyl, alkylene, alkaryl, aryl, or $R_3$-O-$R_4$ groups, with $R_3$ and $R_4$ being independently selected from the group consisting essentially of $C_1$ - $C_{22}$ linear or branched, substituted or unsubstituted alkyl, cycloalkyl, alkylene, alkaryl, and aryl groups under reaction conditions conducive for formation of an imide between the anhydride and the amine, and adding to the imide a source yielding a cation selected from the group consisting essentially of Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives] and mixtures thereof to form a corresponding salt. In certain preferred embodiments of this feature, $R_5$ may be selected from the group consisting essentially of $C_1$ - $C_{18}$ alkyl groups and mixtures thereof and the cation may be $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives] and mixtures thereof. A feature of this embodiment of the invention involves producing a stabile latex composition useful in various pigmented or unpigmented coatings or adhesive systems and which comprises preparing a substantially uniform mixture of ethylenically unsaturated monomers, methacrylate esters, vinyl esters and other known latex forming monomers, along with initiators, base, water and a given amount of a surfactant having the general formula (I) above; and subjecting such mixture to an emulsion polymerization reaction so as to obtain a relatively stabile latex. Yet another feature of this embodiment of the invention involves producing a latex polymeric coating substantially devoid of water-sensitive ingredients and comprises the steps of preparing a relatively stabile latex composition as set forth above, with or without desired pigments, defoamers, antimicrobial agents, etc.; applying a coating of such latex to a surface and subjecting the coated surface to drying conditions including a temperature in the range of about 10° to about 300°C for a period of time sufficient to chemically change the surfactant within said coating to render such latex polymeric coating substantially water-insensitive. A somewhat similar feature of this embodiment of the invention involves producing an adhesive coating substantially devoid of water-sensitive ingredients by preparing a relatively stabile latex composition as set forth above, with or without various other known ingredients, such as alcoholic solvents, defoamers, antimicrobial agents, etc., applying a coating of the adhesive mass to a surface and subjecting the coated surface to drying conditions including a temperature range of about 10° to about 300°C for a period of time sufficient to chemically change the surfactant within the coating to render the adhesive coating substantially water-insensitive. In a particularly preferred embodiment of this feature of the invention, a latex polymerization surfactant (which may be utilized in protective coating systems, adhesive systems, etc.) may be prepared by contacting phthalic anhydride with at least a tertiary $C_1$ - $C_{40}$ amine with stirring so as to obtain an admixture of the anhydride and the amine, and adding a primary or secondary ($R_5R_6NH$) amine to the admixture, with $R_5$ and $R_6$ being independently selected from the group consisting essentially of H or $C_1$ - $C_{40}$ linear or branched, substituted or unsubstituted alkyl, cycloalkyl, alkylene, alkaryl, aryl, or $R_3$-O-$R_4$ groups, with $R_3$ and $R_4$ being independently selected from the group consisting essentially of $C_1$ - $C_{22}$ linear or branched, substituted or unsubstituted alkyl, cycloalkyl, alkylene, alkaryl, aryl groups and mixtures thereof, under reaction conditions conducive for formation of an alkylphthalamate salt. In certain preferred embodiments of this feature of the invention, $R_5$ and $R_6$ in the above amine may be independently selected from a group consisting essentially of H or $C_1$ - $C_{18}$ alkyl groups and mixtures thereof.

It is yet a further object of the invention to provide a method of transporting relatively high viscosity crude petroleum oil which comprises forming a relatively low viscosity emulsion from a select crude petroleum oil, and an emulsifier having the general formula (I) above, and water, and transporting, as by a pump or truck, such emulsion to a desired location, and, at such a location, subjecting such emulsion to conditions sufficient to chemically change the emulsifier to "deactivate" such emulsifier and thereby break the emulsion and allow ready separation of the oil from the admixture. In certain embodiments of the foregoing feature of the invention, conditions sufficient to chemically change the emulsifier so as to deactivate the same involve heating the emulsion to temperatures of at least about 100°C. In yet certain other embodiments of this feature of the invention, conditions sufficient to chemically change the emulsifier so as to deactivate the same, involve adjusting the pH of the emulsion to a value below about 5. A further preferred embodiment of this feature of the invention includes recovering the chemically changed (deactivated) emulsifier and adjusting the pH of the so-recovered deactivated emulsifier to a value above about 7.5 so as to "reactivate" said emulsifier for further use in transporting crude petroleum oil.

It is a further object of the invention to provide an emulsifier/dispersant for crude petroleum oil, with the emulsifier/dispersant having the general formula (I) above.

It is yet a further object of the invention to provide an improvement in the method of scouring textiles which comprises admixing an amount up to about 50 percent of a wetting agent/emulsifier into a textile scouring bath with the wetting agents/ emulsifier having the general formula (I) above. Somewhat similarly, it is the object of the invention to provide improvements in treating textiles/fabrics wherein emulsifiers/lubricants/softeners/dispersants and the like are utilized, such as in spin-finishing, fiber-finishing, coning, winding, dyeing and the like processes or baths or the like, by adding to the treatment process/bath a surfactant having the general formula (I) above.

It is yet a further object of the invention to provide an improvement in the method of preparing pulp from a lignocellulosic material, such as wood chips, wherein chips or the like are subjected to an alkaline liquor at select temperature, pressure and time conditions to obtain a pulp having a select Kappa number range and comprises adding an amount up to about 10 percent of a surfactant having the general formula (I) above, to the alkaline pulping liquor, discharging the pulping liquor and the at least partially delignified lignocellulosic material and displacing the pulping liquor from the partially delignified material with water or an aqueous liquor to obtain a pulp having the select Kappa number range. Of course, the above surfactants, per se or in an aqueous solution may be sprayed onto wood chips or the like prior to subjecting such chips to the alkaline liquor.

It is also an object of the invention to provide an improvement in the method of producing polyurethane/isocyanurate foam by admixing an amount up to about 10 percent of a surfactant having the general formula (I) above with a polyurethane/isocyanurate foam-forming composition and forming a foam from the admixture.

Other and further features, objects and embodiments of the inventions will be apparent to those skilled in the art from the following description and claims.

## Description of Preferred Embodiments

The invention provides surfactants, methods of producing such surfactants, compositions containing such surfactants and methods of utilizing such surfactants in a variety of technical applications.

In accordance with the principals of the invention, surfactants of the present invention comprise compounds having the following general formula:

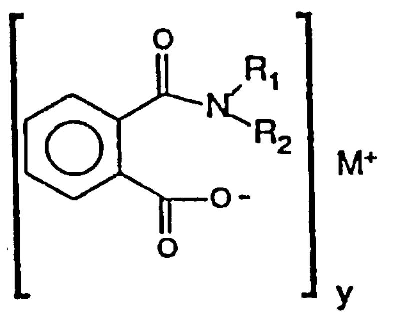

(I)

wherein $R_1$ and $R_2$ are independently selected from the group consisting essentially of H or $C_1$ - $C_{40}$ linear or branched, substituted or unsubstituted alkyl, cycloalkyl, alkylene, alkaryl, aryl or $R_3$-O-$R_4$ groups and mixtures thereof, with $R_3$ and $R_4$ being independently selected from the group consisting of $C_1$ - $C_{22}$ linear or branched, substituted or unsubstituted alkyl, cycloalkyl, alkylene, alkaryl, aryl groups and mixtures thereof; y is an integer of a value satisfying the valency of M; and M is a cation.

Accordingly, the present invention encompasses formulated conditioning shampoos comprising salts of formula I

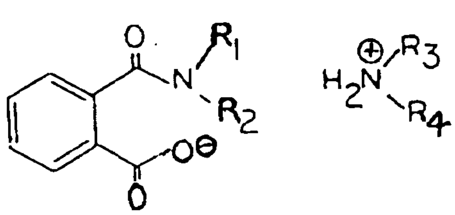

II

wherein

$R_1$, $R_2$, $R_3$, and $R_4$ are the same or different and represent straight or branched chain alkyl groups having 10 to 40 carbon atoms, or aryl straight or branched chain alkyl groups having 10 to 40 carbon atoms.

The present invention also encompasses formulated conditioning shampoos comprising mixtures of a salt of formula II above and an acid of formula III

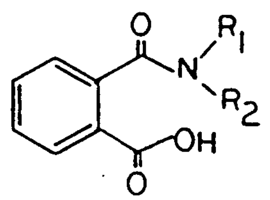

III

wherein

$R_1$ and $R_2$ are the same or different and represent straight or branched chain alkyl groups having 10 to 40 carbon atoms, or aryl straight or branched chain alkyl groups having 10 to 40 carbon atoms.

In a preferred embodiment of the formulated conditioning shampoo of the present invention, $R_1$, $R_2$, $R_3$, and $R_4$ are derived from hydrogenated tallow. Because tallow is a mixture of C-14 to C18 fatty acids, and amines derived from tallow are hence a mixture of tallow amines, the phthalamic acids and/or the ammonium salts thereof used in the present invention may therefore have R groups that are the same or different.

In a particularly preferred embodiment of the formulated conditioning shampoo of the present invention, the shampoo comprises N,N-di(hydrogenated tallow) phthalamic acid di(hydrogenated tallow) ammonium salt. In another particularly-preferred embodiment of the present invention, the shampoo comprises a mixture of N.N-di(hydrogenated tallow) phthalamic acid and N,N-di(hydrogenated tallow) phthalamic acid di-(hydrogenated tallow) ammonium salt.

The effective concentration of these phthalamic acid ammonium salts and mixtures of the phthalamic acids and phthalamic acid ammonium salts in the formulated conditioning shampoos of the present invention varies from about 0.05% to about 20% on an active basis. A presently preferred use concentration appears to be between about 0.5% to 10%. Various formulated shampoos are shown in Tables K1, K2 and K3.

The formulated conditioning shampoos of the present invention comprising a mixture of a salt of formula II and an acid of formula III have ratios of acid to salt varying from about 90:10 to about 10:90. Preferred ratios of acid to salt vary from about 70:30 to about 30:70.

Furthermore, it has been found that the conditioning properties of the shampoos of the present invention are obtained when the pH of the shampoo mixture is adjusted to between about 3.0 and about 9.0. A more preferred range of pH in the formulated shampoos is from about 4.5 to 6.5.

As can be seen from the results shown in Table KA, the shampoo formulations of the invention demonstrated superior overall conditioning attributes, both in a conditioning and conditioning/anti-dandruff formulation. Further, as shown by results in Table KB, the inventive shampoo formulations readily passed freeze/thaw and stability studies over a pH range of 3.0 to 9.0, whereas similar formulations without the di-(hydrogenated tallow) phthalamic acid/ammonium salts of the invention failed.

The formulated conditioning shampoos of the present invention are readily manufactured using a conventional single-phase, hot process. The manufacture of the conditioning shampoos using a single-phase, hot process is simple and, therefore, preferred over multi-phase processes.

The formulated conditioning shampoos of the present invention are prepared by incorporating salts of formula II or a mixture of salts of formula I and acids of formula III in a typical shampoo base such as those shown in Tables K1, K2 and K3.

Certain mixtures of phthalamic acids and phthalamic acid ammonium salts are also excellent emulsifiers/suspending agents for non-volatile and volatile silicones and other water insoluble emollient oils. The utilization of this technology can be naturally extended to car wax formulations, textile lubricants, textile anti-static agents, shoe polishes, antiperspirants, hair conditioners (leave-on or rinse out type) and to other uses of silicone products.

Further, the invention also provides surfactant solutions comprising an effective surfactant amount of salts of formula II

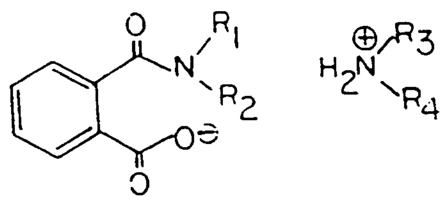

II

wherein

$R_1$, $R_2$, $R_3$, and $R_4$ are the same or different and represent straight or branched chain alkyl groups having 10 to 40 carbon atoms, or aryl straight or branched chain alkyl groups having 10 to 40 carbon atoms.

This invention further provides surfactant solutions comprising an effective amount of a mixture of a salt of formula II above and an acid of formula II

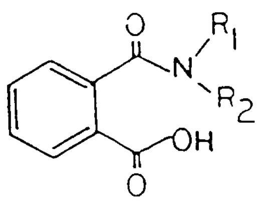

III

wherein

$R_1$ and $R_2$ are the same or different and represent straight or branched chain alkyl groups having 10 to 40 carbon atoms, or aryl straight or branched chain alkyl groups having 10 to 40 carbon atoms.

The surfactant solutions of the present invention may be used as surfactants per se, as caustic-stabile surfactants, as chlorine - stabile surfactants, as cosmetic emulsifiers for personal care products, such as skin creams, skin lotions, hair conditioners, etc., as fast-breaking skin-care product emulsifiers; as emulsifiers for liquid hand, facial and body soaps and bar soaps; as emulsifiers for agricultural chemicals, as domestic fabric softeners; as destructible latex polymerization surfactants in coating and/or adhesive systems, as domestic detergent additives, such as in heavy-duty detergents, in light-duty detergents, in dishwashing detergents, in various hard- surface cleaners, etc., as emulsifiers for portland cement and concrete; as flotation/benefication additives for various mineral ores; as additives for electroplating and/or surface finishing baths for metal goods; as additives for plaster, gypsum and miscellaneous building materials; as enhanced oil recovery additives; as wetting, lubricating, and penetrating surfactants for textile processing; as pulp digestive additives; as surfactants for polyurethane/isocynaurate foam systems; as pour-point depressants for transporting viscous petroleum oils; as industrial surfactants for emulsifying a wide variety of oily materials or oliginous materials such as linseed oils, alkyd resins, polybutylenes, silicones, polysilicones, silicone gums, etc.; as low temperature stabilizers for fatty alcohol/water emulsions; as suspending agents for various particulate material, such as coal tar, sulfur or coal; etc.

Related surfactants and surfactant compositions are disclosed in Goze et al., U.S. Patent Application No. 07/391,187 filed August 8, 1989, and assigned to the same assignee as the instant application. This disclosure is hereby incorporated by reference.

The present invention also provides surfactant solutions comprising a mixture of a salt of formula I and an acid of formula II having ratios of acid to salt ranging from about 90:10 to about 10:90. Preferred ratios of acid to salt range from about 70:30 to about 30:70. The invention also provides surfactant solutions comprising the acid or salt per se.

It has also been unexpectedly found that phthalamic acid ammonium salts function as superior emulsifiers or suspending agents for active agents in anti-dandruff shampoos, such as zinc pyrithione (ZPT) in typical anti-dandruff shampoos. An example of anti-dandruff shampoo with typical phthalamic acid ammonium salt of interest is shown in Table K2 below. Similarly, anti-dandruff shampoo compositions can be formulated with other anti-dandruff agents, such as selenium sulfide, colloidal or powdered sulfur, coal tar mixtures, etc. Certain phthalamic acid ammonium salts of interest also function as emulsifiers and suspending agents in conditioning/antidandruff shampoo compositions.

An example of a conditioning/anti-dandruff shampoo composition with phthalamic acid ammonium salts is shown in Table K3 below. Compositions of this type have been found to provide excellent detangling, wet combability, dry combability, and static control characteristics on hair swatches in a laboratory setting and on human subjects with different hair profiles in a beauty salon setting. These compositions also exhibited excellent long-term stability at various storage temperatures, including three freeze-thaw cycles. The effective concentration of phthalamic acid ammonium salts as a suspending agent varies from about 0.05% to about 20.0% on an active basis. A presently preferred use concentration appears to be between about 0.5% to 10% on an active basis.

Yet further, the invention also provides emulsifier solutions comprising an effective emulsifier amount of salts of formula II

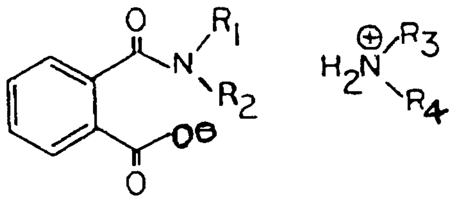

II

wherein

$R_1$, $R_2$, $R_3$, and $R_4$ are the same or different and represent straight or branched chain alkyl groups having 10 to 40 carbon atoms, or aryl straight or branched chain alkyl groups having 10 to 40 carbon atoms.

This invention further provides emulsifier solutions comprising an effective emulsifier amount of a mixture of a salt of formula I above and an acid of formula II

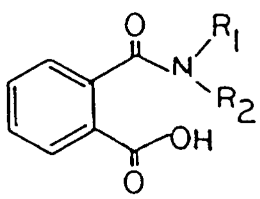

III

wherein

$R_1$ and $R_2$ are the same or different and represent straight or branched chain alkyl groups having 10 to 40 carbon atoms, or aryl straight or branched chain alkyl groups having 10 to 40 carbon atoms.

In certain embodiments of the invention, the $R_1$ and $R_2$ moieties in formula (I) above, are typically hydrocarbon moieties and may include moieties wherein one or more of the carbon atoms in the respective carbon chains are replaced by a hetero atom, such as, for example, oxygen and/or nitrogen. Further, in certain embodiments of the invention the aromatic moiety in the above formulation may be substituted with various functional groups, which include but are not limited to, halides, carboxylic acids and derivatives thereof, nitro, nitroso, amines, amidos, hydroxyls, sulfonic acids and derivatives thereof, alkyls, ethers, esters, nitriles and mixtures thereof.

In certain embodiments of the inventions, the cation, M, in formula (I) above, may have a valency ranging from 1 to about 4 and may be selected from the group consisting essentially of H, Na, K, $NH_4$ - [including $(CH_3CH_2)_3NH$, $(CH_3CH_2)_2NH_2$, $(HOCH_2CH_2)_3NH$, $(HOCH_2CH_2)_2NH_2$ and similar ammonium derivatives], Ba, Ca, Mg, Al, Ti, Zr and mixtures thereof. In certain preferred embodiments of the invention, M may be selected from the group consisting essentially of Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives] and mixtures thereof.

In certain preferred embodiments of the surfactant having formula (I) above, $R_1$ and $R_2$ may be independently selected from the group consisting essentially of H or $C_1$ - $C_{30}$ alkyl groups and more preferrably $C_1$ - $C_{22}$ alkyl groups and M may be selected from a group consisting essentially of Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives] and mixtures thereof.

Specifically preferred embodiments of the surfactants having the formula (I) above, include compounds wherein $R_1$ and $R_2$ may be independently selected from the group consisting essentially of H or $C_1$ - $C_{18}$

alkyl groups and M may be selected from the group consisting essentially of H, Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives]. These preferred compounds have many technical utilities, including as cleansers, emulsifers, wetting agents, lubricants, surfactants, etc., in such diverse applications as caustic-stabile surfactants useful, for example in textile scouring baths or textile spin-finishing operations; chlorine-stabile surfactants useful, for example, in hard-surface cleaners containing bleach, or in liquid automatic dishwashing detergents, or in domestic laundry cleansing formulations containing chlorine, etc.; as foodstuff emulsifiers useful in emulsifying various edible oils and the like into foodstuff compositions, such as baked goods, mayonnaise, dog/cat food, etc.; as emulsifers for skin/hair-care products and useful in emulsifying various emollients or conditioning/protective agents used in treating skin and/or hair; as fabric softeners for treating domestic laundry; as industrial emulsifiers useful in emulsifying various oily materials, such as crude petroleum oils, linseed oils, vitamin acetates, etc., including agricultural chemicals such as oil or water soluble or dispersable pesticides and/or herbicides; as latex polymerization surfactants useful in producing water-insensitive pigment and/or adhesive coatings, etc.; as pulp digestive additives; as polyurethane/polyisocyanurate foam surfactant additives, etc. Exemplary specifically preferred embodiments of the invention include:
sodium N-dodecylphthalamate,
sodium N-octadecylphthalamate,
sodium N-hexadecylphthalamate,
sodium N-tallowphthalamate,
sodium N-cocophthalamate,
sodium N-isododecyloxypropylphthalamate.
N, N- di(hydrogenated tallow) phthalamic acid
N, N- di(Hydrogenated tallow) phthalamic acid di(hydrogenated tallow)-ammonium salt
sodium N-methyl-N-octadecylphthalamate,
sodium N-methyl-N-hexadecylphthalamate,
sodium N-methyl-N-tallowphthalamate,
sodium N-methyl-N-cocophthalamate, mixtures thereof and their corresponding potassium, ammonium, triethylammonium and triethanolammonium salts.

Embodiments of the invention include synthesis routes useful in manufacturing surfactants of formula (I) above at relatively high yields, with little or no purification requirements.

In certain of these embodiments, molten phthalic anhydride may be contacted with at least a primary ($R_5NH_2$) amine, wherein $R_5$ is selected from the group consisting essentially of $C_1$ - $C_{40}$ linear or branched, substituted or unsubstituted, alkyl, cycloalkyl, alkylene, alkaryl, aryl, or $R_3$-O-$R_4$ groups, with $R_3$ and $R_4$ being independently selected from the group consisting essentially of $C_1$ - $C_{22}$ linear or branched, substituted or unsubstituted, alkyl, cycloalkyl, alkylene, alkaryl and aryl groups, under reaction conditions conducive for formation of an imide between the anhydride and the amine, and thereafter adding to the resulting imide, a nucleophilic source yielding an alkali (i.e. Na, K, $NH_4$) cation under reaction conditions conducive to the formation of the corresponding alkali salt. In instances where an alkaline-earth metal salt is desired, a hydroxide of a desired alkaline-earth metal may be added to the above alkali salt solution to form the corresponding alkaline-earth metal (i.e., Ca, Ba or Mg) salt. Somewhat similarly, in instances where an Al, Ti, and/or Zr salt is desired, the alkali salt solution may be acidified, as by addition of a mineral acid, the phthalamic acid isolated and reacted with a $C_1$ - $C_4$ alkoxide of Al, Ti, Zr, or mixtures thereof or at least one $C_1$ - $C_4$ amine may be added to form the corresponding salt. Reaction conditions conducive to formation of the imide and the desired salt generally include applying heat in the range of about 90° to about 150°C while stirring, preferably under an inert atmosphere, such as nitrogen, over a period of time, typically at least about 30 minutes and less than about 300 minutes. During imide formation, water may be removed and during salt formation water may be added.

In certain other of these embodiments, surfactants of formula (I) above may be produced by contacting molten phthalic anhydride with a least a secondary ($R_5R_6NH$) amine wherein $R_5$ and $R_6$ are independently selected from the group consisting essentially of $C_1$ - $C_{40}$ linear or branched, substituted or unsubstituted, alkyl, cycloalkyl, alkylene, alkaryl, aryl, or $R_3$-O-$R_4$ groups, wherein $R_3$ and $R_4$ are defined as above, under reaction conditions conducive for formation of phthalamic acid between the anhydride and the amine, and thereafter adding a source yielding a cation selected from the group consisting essentially of Na, K, $NH_4$ - [including $(CH_3CH_2)_3NH$, $(CH_3CH_2)_2NH_2$, $(HOCH_2CH_2)_3NH$, $(HOCH_2CH_2)_2HN_2$ and similar ammonium derivatives], Ba, Ca, Mg, Al, Ti, Zr and mixtures thereof under reaction conditions conducive for formation of the corresponding salt.

In yet certain other of these embodiments, surfactants of formula (I) above, may be produced by contacting phthalic anhydride with at least a tertiary $C_1$ - $C_{40}$ amine so as to obtain an admixture, typically a substantially uniform admixture, and adding to such admixture a primary or secondary ($R_5R_6NH$) amine, with $R_5$ and $R_6$ being independently selected from the group consisting essentially of H or $C_1$ - $C_{40}$ linear or branched, substituted or unsubstituted, alkyl, cycloalkyl, alkylene, alkaryl, aryl, or $R_3$-O-$R_4$ groups, wherein $R_3$ and $R_4$ are defined as above, under reaction conditions conducive for formation of an alkylphthalamate salt.

In certain reaction schemes, the foregoing steps may occur sequentially or substantially simultaneously.

With the foregoing general discussion in mind, there will now be presented a number of detailed exemplary embodiments of the invention and workers skilled in the art will appreciate that the following examples are non-limitive embodiments of the invention and are included merely as specific exemplification of the invention.

EXAMPLE I

Synthesis of Alkylphthalamate With Primary Amines

A number of alkylphthalamate surfactants are produced from phthalic anhydride and various fatty primary amines in accordance with the following reaction scheme:

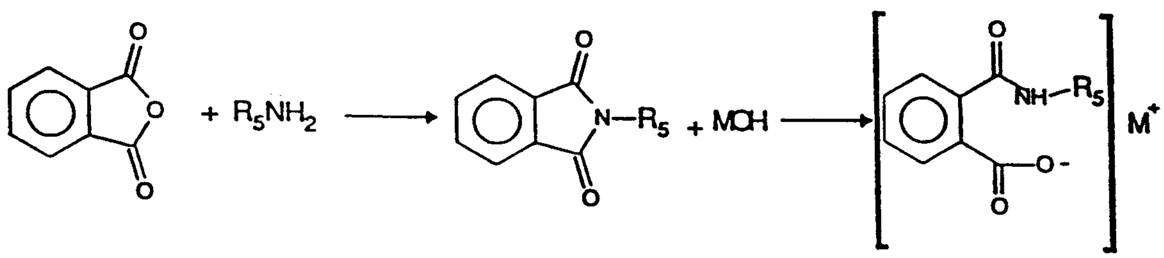

wherein $R_5$ is selected from $C_1$ - $C_{40}$ linear or branched, substituted or unsubstituted alkyl, cycloalkyl, alkylene, alkaryl, aryl, or $R_3$-O-$R_4$ groups, with $R_3$ and $R_4$ being independently selected from $C_1$ - $C_{22}$ linear or branched, substituted or unsubstituted alkyl, cycloalkyl, alkylene, alkaryl, and aryl groups and M is an alkali cation (i.e. Na, K $NH_4$, etc.).

In all synthesis runs of this Example, select reactants may be placed in a suitable reaction flask, which typically may be fitted with a stirrer, and Allihn condenser, a Dean-Stark trap, a controlled inlet interconnected to a nitrogen gas source, a thermometer and an addition funnel, or their equivalent. Phthalic anhydride may be placed in the reactor flask, heated to a melt (about 131 °C), generally under a nitrogen blanket, and an amount of a select fatty amine may be brought into contact with the molten phthalic anhydride. Heating may be continued over a period of time ranging from at least about 30 minutes to about 300 minutes, with removal of water aided by a nitrogen or other inert gas sparge. Thereafter, deionized water may be added and the temperature of the reaction mixture may be adjusted to about 100 °C and a select nucleophilic source, i.e., a hydroxide, yielding a desired cation may be added relatively quickly. This reaction mixture may be heated with stirring for a period of time varying from at least about 30 minutes to about 300 minutes to obtain a high yield, typically about 90% or more, of an alkylphthalamate without requiring purification. Of course, if desired, purification may be effected.

Phthalic anhydride is a well-known chemical and is commercially available, for example from Stepan Company, Northfield, Illinois, (assignee of the present invention). Fatty primary amines are likewise well known and are commercially available from various sources, including, for example, Aldrich Chemical Company.

Exemplary fatty primary amines include: alkyl, cycloalkyl, and/or alkylene amines, such as hexylamine, cyclohexolamine octylamine, decylamine, dodecylamine, tetradecylamine, hexadecylamine octadecylamine, eicosylamine, docosylamine, cocoamine, tallowamine, and similar linear or branched, substituted or unsubstituted alkyl, cycloalkyl or alkylene fatty amines; alkaryl amines, such as 4-methylaniline, 2,4-dimethylaniline, 3,4-diisopropylaniline, 3,5-dimethylaniline, and similar linear or branched, substituted or unsubstituted alkaryl fatty amines; aryl amines, such as aniline, 2-bromoaniline, 2-chloroaniline, 3-bromoaniline, 4-chloroaniline, 3,4-diethylaniline, 3,4,5-tribromoaniline and similar substituted or unsubstituted aryl fatty amines; fatty ether amines, such as isododecyloxypropylamine, isodecyloxypropylamine, hexyloxypropylamine, and similar fatty ether amine compounds.

Exemplary nucleophilic sources yielding alkali cations include: NaOH, KOH, $NH_4OH$. Exemplary sources yielding alkaline-earth metal cation include: $Ca(OH)_2$, $Ba(OH)_2$, $Mg(OH)_2$. Exemplary sources yielding Al, Ti, or Zr cations include: $Al[OCH(CH_3)_2]_3$, $Ti(OCH_2CH_2CH_3)_4$, $Zr(OCH_2CH_2CH_3)_4$ and similar alkoxide compounds.

Specific synthesis of sodium N-octadecylphthalamate, ammonium N-dodecylphthalamate, potassium N-cocophthalamate, sodium N-dodecylphthalamate, sodium N-isododecyloxypropylphthalamate, calcium N-octadecylphthalamate and aluminum N-octadecylphthalamate will now be set forth and workers in the art will recognize that similar syntheses may be performed to attain a desired alkyl, cycloalkyl, alkylene, dialkyl, dialkylene, alkaryl, aryl, etc. phthalamate salt.

## EXAMPLE IA

### Sodium N-Octadecylphthalamate

Phthalic anhydride (1 mole, 148g) was placed in a two liter, three neck, round bottom reaction flask fitted with a magnetic stirrer, an Allihn condenser, a Dean-Stark trap, an inlet for nitrogen, a thermometer and an addition funnel. The phthalic anhydride was heated under a nitrogen blanket to a melt (about 131 °C). Octadecylamine (1 mole, 278g) was added slowly over about 30 minutes to the molten phthalic anhydride. Heating was continued at about 131 °C for about 3 hours, with the nitrogen blanket changed to a nitrogen sparge to aid in the removal of water. A clear pale yellow liquid was obtained, from which a small sample was withdrawn, allowed to solidify (off-white) and characterized by IR spectroscopy (Beckman 983 spectrometer) as comprising N-octadecylphthalimide.

One mole (408g) of this reaction product, N-octadecylphthalimide, was transferred into a twelve liter, 4-neck, round bottom flask fitted with a magnetic stirrer, an Allihn condenser, an addition funnel and an inlet for connection to a nitrogen source (gas cylinder). Deionized water (about 6.1L) was added to the N-octadecylphthalimide and the mixture was heated to about 100 °C to obtain a fairly homogenous mixture. Stirring was then commenced under a nitrogen blanket and 1 N sodium hydroxide (about 1.1L 10% excess) was added relatively quickly over a period of a few minutes. The reaction mixture was stirred with heating for about three hours. A clear light yellow reaction product was obtained and a relatively small sample was removed, dried in a vacuum oven for about 1 hour (1 atm, 40 °C) and characterized by IR spectroscopy as comprising essentially sodium N-octadecylphthalamate.

## EXAMPLE 1B

### Ammonium N-Dodecylphthalamate

The above synthesis is, essentially, duplicated except that dodecylamine is substituted for octadecylamine and ammonium hydroxide is substituted for sodium hydroxide. The final product is characterized by IR spectroscopy as comprised essentially of ammonium N-dodecylphthalamate.

## EXAMPLE 1C

### Potassium N-Cocophthalamate

The synthesis route of Example 1A above is repeated, except that cocoamine is utilized in place of octadecylamine and potassium hydroxide is used in place of sodium hydroxide. The final product is similarly characterized as comprising essentially potassium N-cocophthalamate.

## EXAMPLE 1D

### Sodium N-Dodecylphthalamate

The synthesis route of Example 1A was essentially repeated, except that dodecylamine was utilized in place of octadecylamine. The final product, straw yellow, was characterized by IR spectroscopy as comprising sodium N-dodecylphthalamate. A sample of the reaction product was removed and weighed 52.2980 g as about 11.3 percent solids in water. This sample was quantitatively converted to the corresponding phthalamic acid and using conventional calculations, it was determined that the percent yield of the acid

(or percent purity of the sample) was about 94.16 percent.

EXAMPLE 1E

Sodium N-Isododecyloxypropylphthalamate

The synthesis route of Example 1A was, essentially, repeated except that isododecyloxypropylamine was used in place of octadecylamine and hydrolysis with NaOH occurred at about 70 °C, instead of 100 °C. The final product was characterized by IR spectroscopy as comprising essentially of sodium N-isododecyloxypropylphthalamate.

EXAMPLE 1F

Calcium N-Octadecylphthalamate

A portion of the sodium N-octadecylphthalamate from Example 1A above is acidified with a mineral acid, i.e., hydrochloric acid, to a pH of about 3 and filtered to separate the semi-solid acid. This acid is then admixed with deionized water and a slurry of calcium hydroxide is added, with stirring until a relatively stabile pH of about 8 or 9 is attained. The final product is confirmed by IR spectroscopy as comprising essentially of calcium N-octadecylphthalamate.

Similarly, the calcium salt may also be obtained by adding calcium hydroxide directly to the alkali alkylphthalamate. Also a magnesium or barium salt may be produced by substituting $Mg(OH)_2$ or $Ba(OH)_2$ for the $Ca(OH)_2$ in the above neutralization scheme.

Neutralization of alkyl and/or alkylene phthalamic acids may be accomplished in water or in a non-aqueous solvent, such as methanol, isopropanol, or the like, by addition of a suitable base (including various amines) to obtain a pH of about 8 or 9. When a neat final product is desired, a low-boiling, non-aqueous solvent such as methanol, isopropanol or the like may be used, with subsequent easy removal of the solvent by vacuum stripping.

EXAMPLE 1G

Aluminum N-Octadecylphthalamate

Aluminum isopropoxide (0.0117 moles, 2.38g) and about 200 mL of isopropanol were placed in a 1L four-neck, round bottom reaction flask provided with a nitrogen sparge, an Allihn condenser, a magnetic stirrer and a thermometer. This mixture was heated to a temperature of about 78° C, with stirring for about 1 hour. Then N-octadecylphthalamic acid (0.0353 moles, 15.0g)-obtained via the procedure of Example 1A and acidified with HCl to a pH of about 3 and filtered for separation - was slowly added as a powder. The resultant mixture was heated at reflux (about 78°) for about 3 hours. Upon confirmation of the reaction product by IR spectroscopy, the isopropanol was stripped-off under vacuum. A white solid final product was obtained

EXAMPLE II

Synthesis of Alkylphthalamates With Secondary Amines

A number of alklyphthalamates are produced from phthalic anhydride and various fatty secondary amines in accordance with the following reaction scheme:

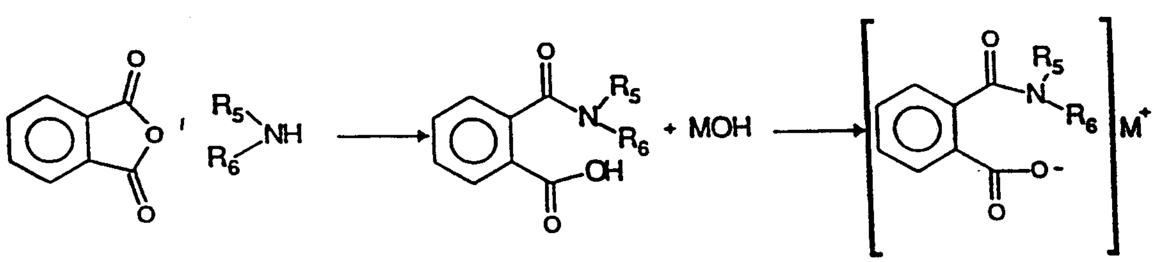

wherein $R_5$ and $R_6$ are independently selected from $C_1$ - $C_{40}$ linear or branched substituted or unsubstituted alkyl, cycloalkyl, alkylene, alkaryl aryl, or $R_3$-O-$R_4$ groups with $R_3$ and $R_4$ being independently selected from $C_1$ - $C_{22}$ linear or branched, substituted or unsubstituted alkyl, cycloalkyl, alkylene, alkaryl, and aryl groups and M is a cation selected from Na, K, $NH_4$ [including $(CH_3CH_2)_3NH$, $(CH_3CH_2)_2NH_2$, $(HOCH_2CH_2)_3NH$, $(HOCH_2CH_2)_2NH_2$ and similar ammonium derivatives], Ba, Ca, Mg, Al, Ti, Zr, and mixtures thereof.

The equipment and conditions for the synthesis runs of this Example are, essentially, identical to those described in Example I above. (Except that the Dean-Stark trap may be eliminated).

Phthalic anhydride is a well known chemical compound and is commercially available, for example, from Stepan Company, Northfield, Illinois (the assignee of the instant invention). Fatty secondary amines are likewise known and are commercially available from various sources, for example, Aldrich Chemical Company.

Exemplary fatty secondary amines include: alkyl, cycloalkyl, and/or alkylene amines, such as; N-dodecyl-N-methylamine, N-tetradecyl-N-methylamine, N-hexadecyl-N-methylamine, N-octadecyl-N-methylamine, N-methyl-N-cyclohexalamine, N-coco-N-methylamine, N-tallow-N-methylamine, N,N-dicocoamine and similar linear or branched, substituted or unsubstituted alkyl, cycloalkyl, or alkylene fatty secondary amines; alkaryl amines, such as; N-methyl-4-dodecylaniline, N-methyl-4-octadecylaniline, N-methyl-4-hexadecylaniline, N-methyl-4-tallowaniline, N-methyl-4-cocoaniline and similar linear or branched, substituted or unsubstituted alkaryl secondary amines; aryl secondary amines, such as: N-methylaniline, N-propylaniline, and similar substituted or unsubstituted and aryl secondary amines.

Exemplary sources yielding select M cations include: NaOH, KOH, $NH_4OH$, $Ca(OH)_2$, $Ba(OH)_2$, $Mg(OH)_2$, $Al[OCH(CH_3)_2]_3$, $Ti(OCH_2CH_2CH_3)_4$, $Zr(OCH_2CH_2CH_3)_4$ and similar sources.

Specific syntheses of sodium N-dodecyl-N-methylphthalamate and triethanolammonium N,N-dicocophthalamate will now be set forth and workers skilled in the art will recognize that similar synthesis routes may be performed to obtain other desired phthalamate salts.

## EXAMPLE IIA

### Sodium N-Dodecyl-N-methylphthalamate

Phthalic anhydride (0.10 mole, 14.8g) was placed in a 500 mL reaction flask similarly configured and equipped to that described in Example 1A The phthalic anhydride was heated to a melt (131 °C) and N-dodecyl-N-methylamine (0.10 mole, 19.9g) was added to the molten anhydride over a period of about 15 minutes. Heating was continued at about 131 °C for about 3 hours. A clear amber, viscous liquid was obtained, which was characterized by IR spectroscopy as N-dodecyl-N-methylphthalamic acid. This reaction product (about 30g) was diluted with methanol (ca. 50g) and neutralized with a methanolic sodium hydroxide (1 N) to a pH of about 8.65. The methanol was then stripped off using a rotary evaporator (water aspirator, 30 °C bath). A white powder was obtained and was characterized by IR spectroscopy as essentially comprising of sodium N-methyl-N-dodecylphthalamate. Methanol was utilized for sake of convenience in producing a 100 percent active material. Water may be utilized if desired to obtain lower active material or if higher temperature, vacuum, etc., are utilized.

## EXAMPLE IIB

### Triethanolammonium N,N-Dicocophthalamate

Phthalic anhydride (0.2 moles, 29.6g) was placed in a reaction flask configured and equipped similarly to that described above in Example IIA, heated to a melt and N, N-dicocoamine (0.2 moles, 76.6 g) was added over a relatively brief time period. Heating was continued at about 131 °C for about 3 hours. A liquid reaction product was obtained and characterized by IR spectroscopy as comprising essentially N,N-dicocophthalamic acid. About 30g of this acid was diluted with water (ca. 50g) and neutralized with triethanolamine to a pH of about 9. The water was then stripped-off as before and the white paste obtained was characterized by IR spectroscopy as comprising essentially triethanolammonium N,N-dicocophthalamate.

When a non-aqueous product is desired, neutralization can occur in a non-aqueous solvent, such as methanol or isopropanol, which can then be removed after neutralization by vacuum.

EXAMPLE III

Synthesis of Alkylphthalamate with Tertiary Amines

A number of alkylphthalamate surfactants may be produced from phthalic anhydride and various fatty primary or secondary amines in the presence of a tertiary amine in accordance with the following reaction scheme:

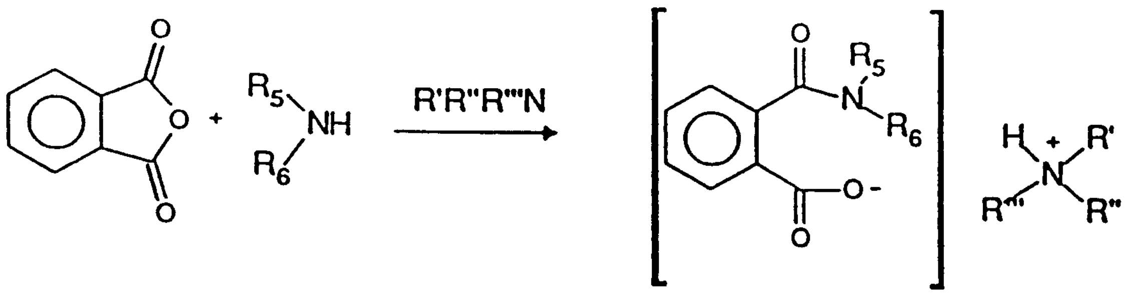


wherein $R_5$ and $R_6$ are independently selected from H or $C_1$ - $C_{40}$ linear or branched, substituted or unsubstituted alkyl, cycloalkyl, alkylene, alkaryl, cyclic including aryl, or $R_3$-O-$R_4$ groups, with $R_3$ and $R_4$ being independently selected from $C_1$ - $C_{22}$ linear or branched, substituted or unsubstituted alkyl, cycloalkyl, alkylene, alkaryl and aryl groups and R', R'' R''' are independently selected from $C_1$ - $C_{40}$ linear or branched, substituted or unsubstituted alkyl, cycloalkyl, alkylene, alkaryl, aryl or $R_3$-O-$R_4$ groups, with $R_3$ and $R_4$ being as defined above.

When a cation other than an ammonium derivative is desired, the above-obtained reaction product may be acidified as set forth earlier, isolated, and then neutralized with a suitable source yielding the desired cation, i.e. Na, K Ca, Ba, Mg, Al, Ti, Zr or mixtures thereof.

The equipment for the synthesis runs of this Example is, essentially, identical to that described earlier, except that an ice bath may be required to control the exothermic nature of this reaction.

As indicated earlier, phthalic anhydride is commercially available from Stepan Company, Northfield, Illinois. Primary and/or secondary amines are likewise known and commercially available from various sources as set forth earlier. Tertiary amines are likewise commercially available.

Exemplary primary and secondary amines are set forth above and exemplary tertiary amines include: triethylamine, triethanolamine, trimethylamine, trimethanolamine, N,N-dimethyl cyclohexylamine, N,N-dimethylaniline and similar alkyl, cycloalkyl, alkylene alkaryl and aryl tertiary amines.

Specific synthesis of triethylammonium N-cocophthalamate, N,N-dimethylcyclohexylammonium N-isododecyloxypropylphthalamate and triethalonammonium N-isododecyloxypropylphthalamate will now be set forth and workers skilled in the art will recognize that similar synthesis routes may be followed to obtain other desired phthalamate salts.

EXAMPLE IIIA

Triethylammonium N-Cocophthalamate

Phthalic anhydride (0.2 moles, 29.6g) and triethylamine (0.2 moles, 20.2g) were introduced into a 500 mL three-neck, round bottom flask, equipped with a magnetic stirrer, Allihn condenser, an inlet for nitrogen, a thermometer and an addition funnel. This mixture was stirred until a fairly uniform mixture was attained. Cocoamine (0.2 moles, 40.8 g) was then slowly added at a rate which maintained the temperature of the reaction mixture below about 35 ° C. After completion of the cocoamine addition, the mixture was stirred for about 1 hour while the temperature of the reaction mixture was maintained at about 30 ° C. A clear yellow liquid product was obtained, cooled to a pale yellow paste and was characterized by IR as essentially comprising triethylammonium N-cocophthalamate.

EXAMPLE IIIA - 1

Triethylammonium N-Cocophthalamate

The procedure of Example IIIA above is repeated except that triethylamine and cocoamine are added substantially simultaneously to the phthalic anhydride. The reaction may be quite exothermic and an ice bath may be utilized to maintain control.

EXAMPLE IIIB

N,N-Dimethylcyclohexylammonium N-Isododecyloxypropylphthalamate

The procedure of Example IIIA was, essentially, repeated, except that N,N-dimethylcyclohexylamine was substituted for triethylamine and isododecyloxypropylamine was substituted for the cocoamine. The final product was characterized by IR spectroscopy as comprising essentially of N,N-dimethylcyclohexylammonium N-isododecyloxypropylphthalamate.

EXAMPLE IIIC

Triethanolammonium N-Isododecyloxypropylphthalamate

The synthesis route of Example IIIA was, essentially, repeated, except that triethanolammonium was substituted for triethylammonium and isododecyloxypropylamine was substituted for the cocoamine. The final product was characterized by IR spectroscopy as comprised essentially of triethanolammonium N-isododecyloxypropylphthalamate.

EXAMPLE A

Foaming and Wetting Characteristics

Standard Ross-Miles Foaming and Draves Wetting tests were preformed on various fatty phthalamates with the results set forth in Table 1 below. In all tests, an active level of 0.1% was utilized.

As may be surmised from the data presented in Table 1, the various sodium alkylphthalamates tested exhibited better foam and wetting characteristics in deionized water than in tap water. It is also apparent that the $C_{12}$ alkyl moiety exhibited the best wetting and foaming characteristics within the alkyl moiety range tested. Further, the alkoxypropylphthalamates appeared to exhibit better wetting characteristics in tap water than in deionized water, with the inverse being true for foaming characteristics. For example, it will be noted that sodium isododecyloxypropylphthalamate exhibited a wetting time of 4.5 seconds with tap water and a foam height of only 5.5 cm. Thus, at least some alkoxypropylphthalamates may have good utility in tap water applications (i.e. domestic laundry detergents, etc.).

TABLE 1

Wetting and Foaming Characteristics of Various Phthalamates

| Compound | Draves (sec) DI* | Tap | Ross-Miles (cm) DI | Tap |
|---|---|---|---|---|
| Sodium N-octylphthalamate | > 120 | > 120 | MMF** | MMF |
| Sodium N-dodecylphthalamate | 6.7 | > 120 | 14.5/14.0 | 6.5/6.0 |
| Triethanolammonium N-dodecylphthalamate | 25.0 | VI*** | 9.5/9.0 | VI*** |
| Sodium N-hexadecylphthalamate | > 120 | > 120 | 6.5/6.5 | MMF |
| Sodium N-octadecylphthalamate | > 120 | > 120 | 7.0/4.0 | MMF |
| Sodium N-cocophthalamate | 21.3 | 54.0 | 10.5/10.5 | 11.8/8.0 |
| Sodium N-methyl-N-dodecylphthalamate | 9.0 | 11.5 | 12.0/11.5 | 4.5/4.0 |
| Sodium N-octyl/decyloxypropyl phthalamate | 33.6 | 25.4 | 7.0/7.0 | 7.5/7.0 |
| Sodium N-Isodecyloxypropylphthalamate | 16.9 | 7.0 | 5.0/4.0 | 7.8/7.0 |
| Sodium N-Isodecyloxypropylphthalamate | 6.2 | 4.5 | 10.5/10.2 | 5.5/5.3 |
| Sodium N-C12-C15 oxypropylphthalamate | 21.5 | 17.1 | 11.0/11.0 | 4.0/3.5 |

*DI = Deionized Water

**NMF = No Measurable Foam

***VI = Very Insoluble

EXAMPLE B

Emulsion Stability Characteristics

In order to obtain reproducible results, an emulsion stability test, modified from the standard used in the art for testing surfactants was developed. In the modified procedure, 0.5g of a select surfactant (instead of 1g) was dissolved in 90 mL of deionized water in a graduated cylinder containing 5.0 mL of xylene, which was then sealed and rocked between a vertical/horizontal position for 30 cycles, allowed to settle for about 5 to 10 minutes and subjected to the same rocking cycle with the results of the second sequence recorded (instead of the first sequence). The results obtained for various alkylphthalamates are set forth in Table II below:

TABLE II

| Compound | Emulsion Break Rate (mL/sec) |
|---|---|
| Sodium N-octylphthalamate | 0.111 |
| Sodium N-decylphthalamate | 0.034 |
| Sodium N-dodecylphthalamate | 0.068 |
| Sodium N-tetradecylphthalamate | 0.085 |
| Sodium N-hexadecylphthalamate | 0.102 |
| Triethanolammonium N-octylphthalamate | 0.043 |
| Triethanolammonium N-decylphthalamate | 0.046 |
| Triethanolammonium N-dodeylphthalamate | 0.039 |

As is apparent from the above data, the triethanolammonium salts exhibited better (i.e., slower) emulsion break rates relative to the sodium salts. However, in personal-care formulations, (which are materially different systems from a xylene/water system) the sodium salts appeared to perform as well or better than the triethanolammonium salts.

EXAMPLE C

Caustic Stability Characteristics

In order to determine caustic stability of the phthalamates, 5g of octylphthalamic acid was dissolved in 450 mL of water containing an equivalent of NaOH. This mixture was heated at reflux for about 24 hours and five separate 90mL samples were withdrawn at 0 hours, 1 hour, 2 hours, 16.5 hours and 23.7 hours. The resulting individual samples were acidified and the sodium N-octylphthalamic acid was isolated and analyzed (filtered, dried, weighted and m.p. and neutralization equivalents were obtained). The results are set forth in Table III below:

TABLE III

| Sample | Time(hr) | Mass(g) | M.P.(°C) | pH | ES*(mL/sec) | Neut.E. |
|---|---|---|---|---|---|---|
| 1 | 0 | 0.77 | 87.5°-88° | 8.70 | 0.0252 | 268 |
| 2 | 1 | 0.69 | 85°-87° | 9.75 | 0.0325 | 296 |
| 3 | 2 | 0.82 | 85°-87° | 10.07 | 0.0400 | 271 |
| 4 | 16.5 | 0.82 | 89.5°-90.5° | 9.96 | 0.0690 | 288 |
| 5 | 23.7 | 0.76 | 87°-89° | 9.88 | 0.108 | 282 |
| | | | | | | Theoretical 277 |

*ES = Emulsion stability

From the foregoing data it is apparent that alkylphthalamates are relatively stabile in a caustic environment and that sodium N-octylphthalamate was stabile at a pH of at least 10.

EXAMPLE D

Thermal Stability Characteristics

Thermal stability studies were conducted with neat (100% actives) sample of ammonium N-dodecylphthalamate mulled on sodium chloride plates. These plates were then placed in an oven heated at about 100 °C for various time periods (0 to 30 minutes) and thereafter characterized by IR spectroscopy. Conversion of a portion of the ammonium N-dodecylphthalamate to the N-dodecylphthalimide was very apparent after only 10 minutes, with almost total conversion to the N-dodecylphthalimide after only 20

minutes. These results are very similar to results obtained for various other alkylammonium alkylphthalamates.

On the other hand, similar studies with sodium alkylphthalamates exhibited a relatively higher degree of thermal stability. For example, sodium N-dodecylphthalamate was stabile at 120 °C over about 28 hours, while sodium N-octadecylphthalamate exhibited only a very small degree of imidation after 3.5 hours at 150 °C. Nevertheless, after 64 hours at 150 °C significant formation of N-octadecylphthalimide was noted. These results show the destructible nature of these surfactants thermally.

EXAMPLE E

Solubility and Temperature Stability Characteristics

Sodium N-octadecylphthalamate, 1% actives, was evaluated for solubility in various solvents and under cold storage (4 °C) and freeze-thaw conditions. The results are summarized in Table IV below.

TABLE IV

| VEHICLE | APPEARANCE 25°C | 50°C | 24 HOURS @ RT° AFTER HEAT | STABILITY COLD STORAGE | FREEZE THAW |
|---|---|---|---|---|---|
| MINERAL OIL | *1 | 1 | 1 | +- | - |
| ISOPROPYL PALMITATE | 1 | 1 | 1 | - | - |
| ISOPROPYL MYRISTATE | 1 | 1 | 1 | - | - |
| ETHANOL | 1 | **S | 1 | - | - |
| $^{1}$NEOBEE$^{R}$ M-5 | 1 | 1 | 1 | - | - |
| $^{2}$NEOBEE$^{R}$ M-20 | 1 | 1 | 1 | - | - |
| $^{3}$NEOBEE$^{R}$ 18 | 1 | 1 | 1 | - | - |
| PETROLATUM | 1 | S | 1 | - | - |
| $^{4}$WECOBEE$^{R}$ S | 1 | 1 | 1 | - | - |
| WATER | 1 | S | 1 | - | - |
| $^{S}$ALPHA-STEP | S | S | S | P | P |
| $^{6}$STEPANOL$^{R}$ WA-EX | 1 | S | S | ***P | P |
| $^{7}$STEOL$^{R}$ CS-330 | 1 | S | S | P | ++P |

## TABLE IV (CONTINUED)

| VEHICLE | APPEARANCE 25°C | 50°C | 24 HOURS @ RT° AFTER HEAT | STABILITY COLD STORAGE | FREEZE THAW |
|---|---|---|---|---|---|
| [8]NINOL[R] 40-CO | I | S | S | P | P |
| [9]AMMONYX[R] LO | S | S | S | P | P |
| [10]AMPHOSOL[R] | I | S | S | P | P |
| [11]MAKON[R] 10 | I | I | S | P | P |
| [12]STEPANATE[R] | I | I | I | • | • |

*I - INSOLUBLE **S - SOLUBLE
***P - PASS ** - FAIL
♦ - not determined due to insolubility

COLD STORAGE - PASS: Returns to clear state after 24 hours at 4°C
FREEZE THAW - PASS: Returns to clear state after 3 freezing/thawing cycles

1 NEOBEE$^R$ M-5 = Stepan Company's registered trademark for caprylic/capric triglyceride.

2 NEOBEE$^R$ M-20 = Stepan Company's registered trademark for propylene glycol dicaprylate/dicaprate.

3 NEOBEE$^R$ 18 = Stepan Company's registered trademark for safflower oil.

4 WECOBEE$^R$ = Stepan Company's registered trademark for hydrogenated vegetable oil.

5 ALPHA-STEP$^R$ ML-40 Stepan Company's registered trademark for sodium alpha-sulfo methyl ethyl laurate.

6 STEPANOL$^R$ WA-EX = Stepan Company's registered trademark for sodium lauryl sulfate.

7 STEOL$^R$ CS-330 = Stepan Company's registered trademark for sodium laureth sulfate.

8 NINOL$^R$ 40-C = Stepan Company's registered trademark for N,N-diethanolcocomide.

9 AMMONYX $^R$LO = Stepan Company's registered trademark for laurylamine oxide.

10 AMPHOSOL$^R$ CG = Stepan Company's registered trademark for cocamide propyl betaine.

11 MAKON$^R$ = Stepan Company's registered trademark for nonylphenol polyethoxylate.

12 STEPANATE$^R$ X = Stepan Company's registered trademark for sodium xylene sulfonate.

## EXAMPLE F

### Bleach (Chlorine Ion) Stability Characteristics

Select N-alkyl and N,N-dialkyl phthalamates were incorporated into a standard test formulation containing a common bleach. Substantially identical formulations were prepared with a commercially available surfactant promoted for bleach stability [1](DOWFAX$^R$-2A1) and with a commercially available surfactant known to exhibit relatively poor bleach stability [2](MAKON$^R$-10), along with a control formulation (no surfactant). The standard formulation comprised 4.0% actives of NaOCl; 0.25% actives NaCl; 1.0% actives NaOH (50%); 2.0% actives surfactant and Q.S. 100% deionized water.

The various formulations were individually placed in tightly capped jars and subjected to a 90°C water bath for about 5 hours and then observed for formulation clarity and foaming characteristics. Each sample was run in duplicate. The control formulation remained clear yellow throughout the test. The MAKON$^R$-10 formulation was initially clear yellow but exhibited clouding and phase separation at about 1.5 hours and showed no foaming after about 5 hours. The DOWFAX$^R$-2A1 formulation was clear yellow throughout the test and exhibited stabile foam after about 5 hours. A formulation containing sodium N-methyl-N-dodecylphthalamate was likewise clear yellow throughout the test but the foam was characterized as unstabile after about 5 hours. On the other hand, a formulation containing sodium N,N-dicocophthalamate exhibited poor solubility, with phase separation and no pre-bath or post-bath foam. Further, a sodium N-dodecylphthalamate formulation was initially clear yellow but became cloudy and exhibited phase separation as the test progressed.

1. DOWFAXR$^R$-2A1 is Dow Chemical Company's registered trademark for sodium branched $C_{12}$ diphenyloxide disulfonate.

2. MAKON$^R$-10 is Stepan Company's registered trademark for a nonylphenol polyethoxylate.

It was concluded from the above that N,N-dialkylphthalamates, particularly sodium N-methyl-N-dodecylphthalamate exhibited good bleach stability and better than that exhibited by the MAKON$^R$ - 10 formulation.

EXAMPLE G

Particulate Material Suspension Characteristics

In this test, potassium N-octadecylphthalamate was utilized as a suspending agent in a typical shampoo base with known particulate anti-dandruff materials, zinc pyrithione and coal tar. Particulate (about 500 mesh) zinc pyrithione and particulate (about 350 to 400 mesh) coal tar were suspended in shampoo base formulations and stored at room temperature, at about 1.6°C and at about 43.3°C. After 5 days the zinc pyrithione formulation exhibited phase separation at 43.3°C with pyrithione settling noted. On the other hand, the coal tar formulation did not show phase separation and coal tar was successfully suspended and exhibited excellent stability after 5 days at room temperature, at 1.6°C and at 43.3°C.

About 2 percent potassium octadecylphthalamate was incorporated with about 50 to 70 percent particulate coal (average size about 200 mesh) and about 30 to 50 percent water at ambient temperature. The resulting admixture was a free flowing, substantially homogeneous suspension which was easily pumped between select locations.

This illustrates that small particle, powdered coal can be fluidized for transport to storage areas or use areas, such as at coal-burning power plants.

EXAMPLE H

Emulsification Characteristics in Foodstuffs

Sodium N-octadecylphthalamate was added to a standard cup-cake recipe at actives levels of 0% (control), 1%, 2%, 3%, 4%, and 5%. The N-octadecylphthalamate was admixed into a typical batter as a partial replacement for the shortening, margarine or butter called for in the recipe, baked at a uniform temperature (about 176°C) and time (about 25 minutes). After cooling, the various cupcakes were examined, with the following results; 0% - tended toward dryness and was crumbly; 1% - appeared and felt moister than the control, retained shape better and exhibited a denser interior; 2% - increased density, more moist; 3% - similar to 2% sample except slightly sunken in middle; 4% - not fully cooked, more sunken in middle; 5% - further cooking required, sunken in middle.

A mayonnaise formulation containing about 1% by weight, based on a 100% total weight of formulation, of sodium N-octadecylphthalamate was prepared, with the N-octadecylphthalamate being substituted for egg yolk in a given recipe. A fluffy creamy white emulsion formed as the salad oil was incorporated into the formulation, which exhibited excellent stability.

A dog food was prepared by admixing about 200g of "Generic" beef-flavored dog food with 3g of 100% actives sodium N-octadecylphthalamate. Samples of this dog food were exposed to various temperatures (i.e., 0°C, 43°C and 50°C). Heated samples, when cooled to room temperature, exhibited no visible oily pockets but after refrigeration for 24 hours and storage for 8 hours at room temperature, showed visible oil pockets. However, after a further 24 hour storage at room temperature these samples showed no oil pockets.

EXAMPLE I

Anionic Emulsification Characteristics for Personal-Care Formulations

Four typical personal-care formulations were parepared with different N-alkylphthalamates. In each instance, the ingredients listed under Part A were combined to produce a substantially uniform mixture with application of heat in the range of about 74°C to 77°C (165°F to 170°F). Similarly, the ingredients listed under Part B were combined with heat so as to obtain a uniform mixture. Thereafter, the Part A mixture was added to the Part B mixture, with good agitation and continued heating to acheive a substantially uniform mixture. The pH of the final mixture was then adjusted with citric acid (although other acids may be utilized) to a pH in the range of aoubt 7.0 to 8.5. The resultant mixture was then allowed to cool to about 35°C (45°F) and evaluated. As can be seen from the data presented in Table V below, these surfactants exhibit excellent emulsion quality and provide excellent performance quality.

TABLE V

| PART A | FORMULA A | FORMULA B | FORMULA C | FORMULA D |
|---|---|---|---|---|
| 1. CARNATION MINERAL OIL | 12.0 | 12.0 | ---- | 9.6 |
| 2. GLYCERYL MONOSTEARATE, PURE | 1.8 | 1.8 | 0.40 | 1.70 |
| 3. PROPYL PARABEN | 0.1 | 0.2 | 0.10 | 0.10 |
| 4. STEARYL ALCOHOL | ---- | 3.0 | 1.0 | 1.70 |
| 5. ISOPROPYL MYRISTATE | ---- | ---- | 10.00 | ---- |
| 6. ALOE VERA OIL | ---- | ---- | ---- | 1.90 |
| PART B | | | | |
| 1. SODIUM N-OCTADECYLPHTHALAMATE | 2.2 | ---- | ---- | 2.10 |
| 2. SODIUM N-HEXADECYLPHTHALAMATE | ---- | 2.20 | ---- | ---- |
| 3. N-TRIETHANOLAMMONIUM OCTADECYLPHTHALAMATE | ---- | ---- | 1.1 | ---- |
| 4. GLYCERINE | ---- | ---- | ---- | 4.8 |
| 5. DEIONIZED WATER | QS 100 | QS 100 | QS 100 | QS 100 |
| 6. METHYL PARABEN | 0.10 | 0.20 | 0.10 | 0.10 |
| EMULSION QUALITY | EXCELLENT | EXCELLENT | EXCELLENT | EXCELLENT |
| PERFORMANCE QUALITY | EXCELLENT | EXCELLENT | EXCELLENT | EXCELLENT |

EXAMPLE J

Rapid Breaking Emulsification Characteristics for Personal-Care Formulations

A number of N-alkylphthalamates were formulated into typical personal-care compositions and evaluated.

The effective usage level of the N-alkylphthalamates in these compositions is about 0.01% to about 10% by weight, with the preferred level of about 1.0% to about 4.0% An effective pH range for these compositions is from about 6 to about 10, with a preferred pH range being between about 7.6 to about 9.5. While $C_1$-$C_{40}$ chain lengths are useful, a preferred chain length is about $C_8$ to $C_{18}$ and a more preferred chain length was found to be $C_{16}$, tallow ($C_{16}$ - $C_{18}$) and $C_{18}$. The respective results are set forth in Tables VI-A through VI-C below.

TABLE VI-A

EFFECT OF CONCENTRATION

| | | FORMULA J-1 % | FORMULA J-2 % | FORMULA J-3 % | FORMULA J-4 % | FORMULA J-5 % | FORMULA J-6 % |
|---|---|---|---|---|---|---|---|
| 1. | WATER, DEIONIZED | QS 100 | QS 100 | QS 100 | QS 100 | QS 100 | QS 100 |
| 2. | METHYL PARABEN | 0.15 | 0.15 | 0.15 | 0.15 | 0.15 | 0.15 |
| 3. | SODIUM N-OCTADECYLPHTHALAMATE | 0.50 | 1.00 | 1.50 | 2.00 | 4.00 | 8.00 |
| 4. | KESSCO[R] ISOPROPLY PALMITATE | 10.00 | 10.00 | 10.00 | 10.00 | 10.00 | 10.00 |
| 5. | KESSCO[R] GLYCERYL MONOSTEARATE PURE | 0.25 | 0.50 | 0.75 | 1.00 | 2.00 | 4.00 |
| 6. | KESSCO[R] CETYL ALCOHOL | 1.75 | 1.75 | 1.75 | 1.75 | 1.75 | 1.75 |
| 7. | PROPYL PARABEN | 0.10 | 0.10 | 0.10 | 0.10 | 0.10 | 0.10 |
| 8. | [2]GLYDANT | 0.25 | 0.25 | 0.25 | 0.25 | 0.25 | 0.25 |
| [3] Evaluation for Rapid Breaking Property | | No Emulsion No Evaluation | 26 Sec. | 26 Sec. | 27.5 Sec. | 27.5 Sec. | 37 Sec. |
| Skin Feel | | .... | Light | Light | Light | Light | Light |
| Emollient/Emulsifer Ratio | | 10:0.75 | 10:1.5 | 10:2.25 | 10:3 | 10:6 | 10:12 |

1. KESSCO is a registered trademark of Stepan Company for various organic esters.
2. DMDM Hydontain (preservative).
3. Skin area washed with soap and water, dried; 0.1g of formulation applied; rubbed until dry; time recorded from initial rubbin until non-tacky with no drag

TABLE VI-B

EFFECT OF pH

| | | % | FORMULA J-7 pH = 5.8 | FORMULA J-8 pH = 6.7 | FORMULA J-9 pH = 7.7 | FORMULA J-10 pH = 9.5 |
|---|---|---|---|---|---|---|
| 1. | WATER, DEONIZED | QS 100 | | | | |
| 2. | METHYL PARABEN | 0.15 | NO | NO | STABLE | STABLE |
| 3. | SODIUM N-OCTADECYLPHTHALAMATE | 2.00 | EMULSION | EMULSION | EMULSION | EMULSION |
| 4. | [1]KESSCO$^{R}$ ISOPROPYL PALMITATE | 1.00 | | | | |
| 5. | KESSCO$^{R}$ GLYCERYL MONOSTEARATE PURE | 1.00 | | | | |
| 6. | KESSCO$^{R}$ CETYL ALCOHOL | 1.75 | | | | |
| 7. | PROPYL PARABEN | 0.10 | | | | |
| 8. | [2]GLYDANT | 0.25 | | | | |
| BREAKDOWN TIME | | | ---- | ---- | 26 SEC | 26 SEC |

1. Ibid
2. Ibid

TABLE VI-C

EFFECT OF CHAIN LENGTH

| | FORMULA J-11 | FORMULA J-12 | FORMULA J-13 | FORMULA J-14 | FORMULA J-15 |
|---|---|---|---|---|---|
| 1. WATER, DEONIZED | QS 100 | QS 100 | QS 100 | QS 100 | QS 100 |
| 2. METHYL PARABEN | 0.15 | 0.15 | 0.15 | 0.15 | 0.15 |
| 3. SODIUM N-OCTADECYLPHTHALAMATE | 2.00 | ---- | ---- | ---- | ---- |
| 4. SODIUM N-HEXADECYLPHTHALAMATE | ---- | 2.00 | ---- | ---- | ---- |
| 5. SODIUM N-DODECYLPHTHALAMATE | ---- | ---- | 2.00 | ---- | ---- |
| 6. SODIUM N-OCTYLPHTHALAMATE | ---- | ---- | ---- | 2.00 | ---- |
| 7. SODIUM N-OCTADECYLPHTHALAMATE (TALLOW) | ---- | ---- | ---- | ---- | 2.00 |
| 8. [1]KESSCO$^R$ ISOPROPYL PALMITATE | 10.00 | 10.00 | 10.00 | 10.00 | 10.00 |
| 9. KESSCO$^R$ GLYCERYL MONOSTEARATE PURE | 1.00 | 1.00 | 1.00 | 1.00 | 1.00 |
| 10. KESSCO$^R$ CETYL ALCOHOL | 1.75 | 1.75 | 1.75 | 1.75 | 1.75 |
| 11. PROPYL PARABEN | 0.10 | 0.10 | 0.10 | 0.10 | 0.10 |
| 12. [2]GLYDANT | 0.25 | 0.25 | 0.25 | 0.25 | 0.25 |
| SHELF-LIFE STABILITY | STABLE EMULSION | STABLE EMULSION | NO EMULSION | NO EMULSION | STABLE EMULSION |
| BREAKDOWN TIME | 26 SEC | 26 SEC | ---- | ---- | 27 SEC |

1. Ibid
2. Ibid

Various commercial personal-care products (skin-care lotions) suggest that their formulations provide quick penetration into skin areas brought into contact therewith. It is known in the emulsion technology art that the faster breaking emulsions are oil-in-water, nonionic or anionic soap-based emulsions which contain Carbopol$^R$ (a registered trademark of B.F. Goodrich Company for certain polymerized vinyl resins, see EPA 026816A2) as the primary emulsifier. Carbopol$^R$ materials appear unstabile in the presence of salts

(electrolytes) so that when emulsion products containing such materials are applied to skin, which includes salt from perspiration or the like, the emulsion breaks and emollients and the like, in the product then quickly penetrate the skin. Emulsions without Carbopol$^R$, regardless of type (anionic or nonionic), do not break down as readily when applied to skin.

The N-alkylphthalamates of the invention comprise excellent cosmetic emulsifiers and are water dispersable (i.e., high HLB) emulsifiers. Hydrophilic-lipophilic balance or HLB is a widely accepted measure of the polarity of a surfactant and of its relative affinity for aqueous or hydrocarbon media. It is generally held [see, for example, Nonionic Surfactants, edited by Schick (Marcel Dekker Inc., N.Y. 1967) pages 606-608] that HLB ranges for different surface active functions are as follows:

| | |
|---|---|
| Water-in-oil emulsifier | 3 - 6 |
| Wetting Agent | 7 - 9 |
| Oil-in-water emulsifier | 8 - 15 |
| Detergent | 13 - 15 |
| Solubilizer | 15 - 18 |

The N-alkylphthalamates of the invention are characterized as being relatively high HLB emulsifiers or primary emulsifiers having an HLB value in the range of about 7 to 15, and when utilized to formulate cosmetic emulsions are combined with secondary or relatively low HLB emulsifiers, such as glyceryl stearate to produce rapidly breaking, loosely emulsified emulsions. The resulting optimal emulsions were found to exhibit excellent shelf-life stability while breaking down extremely quickly when applied to skin. As shown in Table VI-D below, emulsion compositions containing a N-alkylphthalamate broke substantially quicker than various commercial products tested.

TABLE VI-D

| EVALUATION OF BREAKDOWN PROPERTY FOR VARIOUS PRODUCTS | |
|---|---|
| PRODUCT TESTED | BREAKDOWN TIME* (SEC) |
| FORMULATION J-11* | 27 |
| [1]VASELINE INTENSIVE CARE LOTION$^R$ | 37 |
| [2]SOFT SENSE LOTION$^R$ | 52 |
| [3]KERI LOTION$^R$ | 47 |
| [4]VISIBLE DIFFERENCE LOTION$^R$ | 52 |
| [5]OIL OF OLAY$^R$ | 43 |
| These tests were performed in triplicate and comprised washing select skin area with soap and water, drying the washed skin area, applying; 0.1g of formulation; rubbing until dry; and recording the elapsed time from initial rubbing until non-tacky with no drag observed. | |

*See Table VI-C
1. Registered trademark of Chesebrough Pond's Inc.
2. Registered trademark of S.C. Johnson & Son, Inc.
3. Registered trademark of Westwood Pharmaceuticals, Inc.
4. Registered trademark of Elizabeth Arden, Inc.
5. Registered trademark of Olay Company, Inc.

Personal care product compositions (such as skin-care lotions or creams) containing the surfactants of the invention exhibit excellent salt tolerance (i.e., up to about 3% of sodium chloride can be incorporated into select compositions without breaking the emulsions).

Further, typical emulsion compositions exhibit excellent solvent tolerance. Stabile emulsion compositions having up to 20% SD-40 alcohol and/or SD-3A alcohol have been successfully prepared.

The N-alkylphthalamate surfactants of the invention can be incorporated into personal-care product compositions via an oil phase or a water phase. Final emulsions are preferably prepared using a normal two phase hot process.

A wide variety of personal-care emollients, such as petrolatum light, mineral oil, isopropyl palmitate, caprylic/capric triglyceride, avacado oil, sesame seed oil, safflower oil, corn oil, peanut oil, lanolin AAA

deodorized, acetylated lanolin, melanin, a variety of sunscreens and vitamins, etc., are readily emulsified by a combination of the relatively high HLB emulsifiers of the invention with a relatively low HLB emulsifier, such as glycerol mono-stearate and a cosmetic emollient of choice so as to attain a ratio of emollient to emulsifier in the range of about 3:1 to about 30:1.

EXAMPLE K

Hair Conditioning Characteristics

Sodium N-octadecylphthalamate was incorporated at 5%, 3% and 1%, respectively, into a standard hair shampoo formulation. These formulations comprised 12% active ammonium lauryl sulfate, 2.5% N,N-diethanol cocoamide and Q.S 100 deionized water, along with the above N-alkylphthalamate. These three hair shampoo compositions were then evaluated on hair swatches by a panel for wet compatibility and dry compatibility, along with three commercially available hair shampoo formulations. Each swatch was rated from 1 (worst) to 5 (best) and the average results are set forth below:

| FORMULATION | WET COMBING | DRY COMBING |
|---|---|---|
| 5% PHTHALAMATE | 4.28 | 3.72 |
| 3% PHTHALAMATE | 2.28 | 3.44 |
| 1% PHTHALAMATE | 3.67 | 3.17 |
| [1]PERT PLUS[R] | 3.70 | 3.70 |
| [2]PRELL[R] | 1.80 | 1.98 |
| [3]SUAVE GOLDEN[R] | 1.39 | 2.64 |

1. Pert Plus[R] is a registered trademark of Procter and Gamble Company for a hair conditioning shampoo.
2. Prell[R] is a registered trademark of Procter and Gamble Company for hair cleaning shampoo.
3. Suave Golden[R] is a registered trademark of Helene Curtis, Inc. for a hair cleaning shampoo.

As can be seen from the above data the 5% phthalamate formulation readily outperformed all three commercial shampoos. Additionally, the 3% and 1% phthalamate formulations outperformed at least two of the commercial shampoos.

TABLE K1

| CONDITIONING SHAMPOO | | |
|---|---|---|
| Ingredient | Formulation 1 wt. % (active) | Formulation 2 wt. % (active) |
| 1. Deionized Water | Q.S. to 100.00 | Q.S. to 100.00 |
| 2. Tetrasodium EDTA | 0.20 | 0.20 |
| 3. STEPANOL® AM-V[1] | 20.00 | 20.00 |
| 4. NINOL® 40 CO[2] | 2.00 | 2.00 |
| 5. N,N-di(hydrogenated tallow) Phthalamic acid/ammonium salt at a ratio of 80:20 | - | 5.00 |
| 6. Silicone DC 200 (12,500 cps) (a dimethicone) | 0.50 | 0.50 |
| 7. Citric Acid 50% | Q.S. | Q.S. |
| 8. Sodium Hydroxide 50% | Q.S. | Q.S. |
| 9. 1,3,5,5-tetramethyl hydantoin | 0.20 | 0.20 |
| 10. Ammonium Chloride | 0.20 | 0.20 |

[1] Registered trademark of Stepan Co. for ammonium lauryl sulfate
[2] Registered trademark of Stepan Co. for cocomide diethanolamine

TABLE K2

| ANTI-DANDRUFF SHAMPOO | | |
|---|---|---|
| Ingredient | Formulation 3 wt. % (active) | Formulation 4 wt. % (active) |
| 1. Deionized Water | Q.S. to 100.00 | Q.S. to 100.00 |
| 2. Tetrasodium EDTA | 0.20 | 0.20 |
| 3. STEPANOL® AM-V[1] | 20.00 | 20.00 |
| 4. NINOL® 40 CO[2] | 2.00 | 2.00 |
| 5. N,N-di(hydrogenated tallow) Phthalamic acid/ammonium salt at a ratio of 30:70 | - | 5.00 |
| 6. Zinc Pyrithione 48% Dispersion | 4.20 | 4.20 |
| 7. Citric Acid 50% | Q.S. | Q.S. |
| 8. Sodium Hydroxide 50% | Q.S. | Q.S. |
| 9. 1,3,5,5-tetramethyl hydantoin | 0.20 | 0.20 |
| 10. Ammonium Chloride | 0.20 | 0.20 |

[1] Registered trademark of Stepan Co. for ammonium lauryl sulfate
[2] Registered trademark of Stepan Co. for cocomide diethanolamine

TABLE K3

| CONDITIONING/ANTI-DANDRUFF SHAMPOO | | |
|---|---|---|
| Ingredient | Formulation 5 wt. % (active) | Formulation 6 wt. % (active) |
| 1. Deionized Water | Q.S. to 100.00 | Q.S. to 100.00 |
| 2. Tetrasodium EDTA | 0.20 | 0.20 |
| 3. STEPANOL® AM-V[1] | 20.00 | 20.00 |
| 4. NINOL® 40 CO[2] | 2.00 | 2.00 |
| 5. N,N-di(hydrogenated Tallow) phthalamic acid/ ammonium salt at a ratio of 50:50 | - | 5.00 |
| 6. Silicone DC 200 (12,500 cps) (a dimethicone) | 0.50 | 0.50 |
| 7. Zinc Pyrithione 48% Dispersion | 4.20 | 4.20 |
| 8. Citric Acid 50% | Q.S. | Q.S. |
| 9. Sodium Hydroxide 50% | Q.S. | Q.S. |
| 10. 1,3,5,5-tetramethyl hydantoin | 0.20 | 0.20 |
| 11. Ammonium Chloride | 0.20 | 0.20 |

[1] Registered trademark of Stepan Co. for ammonium lauryl sulfate
[2] Registered trademark of Stepan Co. for cocomide diethanolamine

TABLE KA

| COMPARATIVE PERFORMANCE EVALUATION - CONDITIONING SHAMPOO AND CONDITIONING/ANTI-DANDRUFF SHAMPOO | | |
|---|---|---|
| CONDITIONING* ATTRIBUTES | CONDITIONING SHAMPOO | CONDITIONING/ANTI-DANDRUFF SHAMPOO |
| Detangling | Form. 2 > Form. 1 > Baseline control | Form. 6 > Form. 5 > Baseline control |
| Wet Combing | Form. 2 > Form. 1 > Baseline control | Form. 6 > Form. 5 > Baseline control |
| Dry Combing | Form. 2 > Form. 1 > Baseline control | Form. 6 > Form. 5 > Baseline control |
| Static Control/Flyaway | Form. 2 > Form. 1 -Baseline control | Form. 6 > Form. 5 -Baseline control |
| Baseline control Formulation is substantially the same as formulations 1 and/or 5 without silicone DC 200 (12,500 cps) | | |

*A controlled half-head salon evaluation by an experienced licensed beautician on a panel of 30 subjects was used as a method for determining the listed attributes above.

TABLE 4B

| COMPARATIVE STABILITY EVALUATION | | | | | | | | |
|---|---|---|---|---|---|---|---|---|
| Formula # | | Temp. (°F) over 1 Month Period | | | | pH over 1 Month Period | | |
| | | -32° | +35° | Ambient | 110° | 3.0 | 7.0 | 9.0 |
| Baseline | | P | S | S | S | S | S | S |
| Control Formula | 1 | U | U | U | U | U | U | U |
| | 2 | P | S | S | S | S | S | S |
| | 3 | U | U | U | U | U | U | U |
| | 4 | P | S | S | S | S | S | S |
| | 5 | U | U | U | U | U | U | U |
| | 6 | P | S | S | S | S | S | S |
| P - Pass (3) Freeze/Thaw cycles<br>S - Stable<br>U - Unstable | | | | | | | | |

EXAMPLE K1

Preparation of a mixture of N,N-di(hydrogenated tallow). phthalamic acid and N,N-di(hydrogenated tallow) phthalamic acid di-(hydrogenated tallow) ammonium salt

One mole (148.0g) of flaked phthalic anhydride (PA) was charged into a 4-neck 5-liter round bottomed flask reactor equipped with a mechanical stirrer, a thermocouple temperature controller and a heating mantle. A charge of 650g of isopropyl alcohol (USP grade) was added to the reactor to achieve a slurry of about 50% solids. One mole (about 502g) of molten di(hydrogenated tallow) amine was slowly added to the slurry in the reactor with continuous stirring. The temperature of the reaction mass was allowed to stabilize at about 45-55°C with gradual addition of the amine and cooling of the reactor flask. Amine addition was completed in about 0.5 to 1 hours. Thereafter the reactor was maintained at about 60°C until the PA flakes dissipated and IR spectroscopic analysis showed no detectable amounts of PA in the reaction mass. Isopropyl alcohol was then removed under vacuum (about 1-50 mmHg) On analysis, about 62 mole % acid and about 38 mole % salt were found with an average molecular weight of about 782.

EXAMPLE K2

Preparation of a Conditioning Shampoo

Water was first added into a suitable vessel equipped with agitation, heating and cooling capabilities. While the water was agitated and heated slowly, tetrasodium EDTA, STEPANOL® AM-V (ammonium lauryl sulfate) and NINOL® 40 CO (cocomide diethamolamine) were added. At about 145 °F a 70:30 mixture of N, N-di(hydrogenated tallow) phthalamic acid and N,N-di(hydrogenated tallow) phthalamic acid N,N-di-(hydrogenated tallow) ammonium salt, followed by silicone DC 200 were added to the mixture. The mixture was heated to 160-165 °F and emulsified for 20-30 minutes at high speed while maintaining the temperature between 160-165 °F. The mixture was slowly cooled with agitation set at medium speed. When the temperature of the mixture cooled to about 110 °C, 1,3,5,5-tetramethyl hydantoin and ammonium chloride were added. The pH was checked and adjusted as necessary with ammonium hydroxide or citric acid to a value between about 4.5 to 6.5. The viscosity was checked and adjusted as necessary with ammonium chloride to a value between 4000 and 6000 cps.

EXAMPLE K3

Preparation of an anti-dandruff shampoo

Water was first added into a suitable vessel equipped with agitation, heating and cooling capabilities. While the water was agitated and heated slowly, tetrasodium EDTA, STEPANOL® AM-V, (ammonium lauryl sulfate) and NINOL® 40 CO, (cocomide diethanolamine) were added. At about 145 °F, a 70:30 mixture of N, N-di(hydrogenated tallow) phthalamic acid and N,N-di(hydrogenated tallow) phthalamic acid N,N-di-(hydrogenated tallow) ammonium salt, followed by silicone DC 200 and ZPT (zinc pyrithione, 48% dispersion), were added to the mixture. The mixture was heated to 160-165 °F and emulsified for 20-30 minutes at high speed while maintaining the temperature between 160-165 °F. The mixture was slowly cooled with agitation set at medium speed. When the temperature of the mixture cooled to about 110 °C, 1,3,5,5-tetramethyl hydantoin and ammonium chloride were added. The pH was checked and adjusted as necessary with ammonium hydroxide or citric acid to a value between about 4.5 and 6.5 The viscosity was checked and adjusted as necessary with ammonium chloride to a value between 4000 and 6000 cps.

EXAMPLE K4

Preparation of an Anti-dandruff/Conditioning Shampoo

Water was first added into a suitable vessel equipped with agitation, heating and cooling capabilities. While the water was agitated and heated slowly, tetrasodium EDTA, STEPANOL® AM-V, (ammonium lauryl sulfate), and NINOL® 40 CO (cocomide diethanolamine) were added. At about 145 °F, a 70:30 mixture of N, N-di(hydrogenated tallow) phthalamic acid and N,N-di(hydrogenated tallow) phthalamic acid N,N-di-(hydrogenated tallow) ammonium salt, followed by silicone DC 200 and ZPT (zinc pyrithione, 48% dispersion) were added to the mixture. The mixture was heated to 160-165 °F and emulsified for 20-30 minutes at high speed while maintaining the temperature between 160-165 °F. The mixture was slowly cooled with agitation set at medium speed. When the temperature of the mixture cooled to about 110 °C, 1,3,5,5-tetramethyl hydantoin and ammonium chloride were added. The pH was checked and adjusted as necessary with ammonium hydroxide or citric acid to a value between about 4.5 and 6.5. The viscosity was checked and adjusted as necessary with ammonium chloride to a value between 4000 and 6000 cps.

As will be appreciated, the above formulations provide exemplary surfactants and cocomides only, and other primary surfactants, amides and mixtures thereof may be

EXAMPLE L

Latex Polymerization Surfactant Characteristics

Latex or emulsion polymers, typically derived from ethylenically unsaturated monomers, methacrylate esters, vinyl esters and similar known latex-forming monomers are widely used in a variety of applications, such as binders for-pigmented or unpigmented protective paints or coatings, as adhesive masses, binders for paper coatings and for non-woven textiles, binders for carpet backing materials and plastisols, binders

for synthetic rubbers, as concrete or portland cement additives, printing ink additives, floor polish and similar wax additives and in other similarly related areas. Most conventional emulsion (latex) polymers are produced by an emulsion polymerization process wherein select monomeric materials are polymerized while they are dispersed in an aqueous medium via surfactants. Such surfactants may be anionic in nature, such as soaps or various sulfates or sulfonates of various organic substances. Alternatively, such surfactants may be nonionic, such as ethylene oxide derivatives of organic substances or cationic, such as alkyl ammonium halides. Further, the polymerization reactions frequently are effected in the presence of water-soluble protective colloids or stabilizing agents. Any one of the above emulsifying or stabilizing agents may lead to the presence of a water-sensitive material in the vinyl polymeric latex, which in many instances is highly undesirable. The art has long theorized that a means of avoiding the presence of water-sensitive materials in a polymeric latex mass is to employ a fugitive or destructible surfactant. Such as surfactant would function as an emulsifier and solubilizer during the polymerization process and as a stabilizer for the latex produced therefrom. Drying of such a latex, as in producing a latex coating, especially forced drying, would drive off or somehow chemically destroy or change the surfactant and render the latex water-insensitive. To date, necessary surface active properties have been found only in molecules too large to be volatile so that destruction of the surfactant appears to be preferred. Some workers in this art (i.e. see U.S. Patents 4,544,726 and/or 3,928,423) suggested surfactants which are incorporated into the polymeric mass during the polymerization process so as to render them incapable of migrating to the surface of the polymeric mass and thereby reducing the water-sensitivity of such polymeric mass. However, none of these or similar materials have been completely satisfactory, largely due to the fact that all remain water-sensitive, even as bound components in the polymeric mass.

As indicated earlier, the invention provides a new and useful class of latex polymerization surfactants having the general formula (I) above and which are readily destroyed or chemically changed during application (coating/heating) so as to render latex compositions containing such substantially water-insensitive. In preferred embodiments of the foregoing surfactant, $R_1$ and $R_2$ may be independently selected from the group consisting essentially of H or $C_1$ - $C_{18}$ alkyl or $R_3$-O-$R_4$ groups and mixtures thereof, with $R_3$ and $R_4$ being independently selected from the group consisting essentially of $C_1$ - $C_{22}$ linear or branched, substituted or unsubstituted alkyl, cycloalkyl, alkylene, alkaryl, aryl groups and mixtures thereof, with the cation, M, being selected from alkali metals (including ammonium and various amine derivatives) and alkaline earth metals. In even more preferred embodiments, $R_1$ and $R_2$ may be independently selected from the group consisting essentially of H or $C_1$ - $C_{18}$ alkyl moieties and M may be selected from ammonium or an amine derivative thereof which boils below about 125°C. An exemplary preferred surfactant comprises triethylammonium N-cocophthalamate.

It is theorized and generally confirmed by infrared (IR) spectroscopy that the latex polymerization surfactants of the invention, once subjected to drying conditions, including a temperature in the range of about 10° to about 300°C for a period of time undergo a chemical change and form an imide, which is substantially water-insensitive and does not exhibit appreciable surface activity.

A number of exemplary latex compositions were prepared and subjected to a water resistance test which involved dipping two microscope slides into each latex composition and allowing the so-coated slides to dry at ambient conditions for 24 hours, while suspended in a vertical position. One of each pair received no further treatment while the other slide of each pair was baked for about 15 minutes in a circulating air oven maintained at about 150°C and allowed to cool to room temperature. All the slides were then immersed in deionized water for a period of time of not less than three weeks and observed for adhesive failure (blistering, pulling away from the glass surface, etc.). The results are tabulated below in Table VII.

Latex composition #1 was prepared by charging into a suitably set-up reactor 205.5 g of deionized water, 53.2 g of dimethylethanolammonium N-dodecylphthalamate (7.52% solids) and 2.0 g of $NH_4OH$ and heated to about 70°C with stirring under a nitrogen blanket. Thereafter, about 20% of a total monomer charge and all of an initiator charge (monomer charge comprised of 100.0 g of butyl acrylate and 100 g of methylmethacrylate; initiator charge comprised of 0.2g of $K_2S_2O_8$ and 20.0 g of deionized water) were added to the reactor and allowed to react. Next, the remainder of the monomer charge was added continuously over about 1 hour along with a separate, continuous and concurrent addition of a feed comprised of 30.0 g of deionized water, 0.8 g of $K_2S_2O_8$ and 2.0 g of $NH_4OH$, with continued beating at about 70° to about 75°C. This polymerization reaction mixture was heated for an additional 1 hour at about 70° to 75°C with stirring. Thereafter, the finished and relatively stabile latex composition was cooled to ambient temperature and discharged for storage and testing.

Latex compositions #2 was essentially similar to composition #1 above and was prepared in a substantially identical matter, except that triethylammmonium N-dodecylphthalamate (about 5.44% solids) was used in place of the dimethylethanolammonium N-dodecylphthalamate, along with minor adjustments of

water content.

Latex composition #3 was prepared in an essentially similar manner, of substantially identical materials as that described in composition #1 except that styrene was used in place of the methylmethacrylate monomer.

Latex composition #4 was prepared in a substantially identical manner and with substantially identical materials to that of composition #2, except that styrene was used in place of the methylmethacrylate.

Latex compositions #5, #6 and #7 were prepared in a substantially identical manner and with substantially identical ingredients as described for composition #1, except that in each respective formulation, sodium lauryl ether sulfate containing 30 moles of ethylene oxide; sodium lauryl ether sulfate containing 4 moles of ethylene oxide and sodium lauryl ether sulfate containing 12 moles of ethylene oxide were utilized in place of the dimethylethanolammmonium N-dodecylphthalamate.

Latex composition #8 was prepared by charging into a suitably set-up reactor 49.0 g of deionized water, 1.5 g of $K_2S_2O_8$ and 2.0 g of triethylamine and heated to about 80°C with stirring under a nitrogen blanket. A pre-emulsion composition comprised of 280.4 g of triethylammonium N-dodecylphthalamate, 2.0 g of triethylamine, 180.0 g of 2-ethylhexylacrylate, 90.0 g of methylmethacrylate and 30.0 g of hydroxyethylacrylate, and an initiator solution comprised of 0.5 g of $K_2S_2O_8$ and 25.0 g of deionized water were made-up and each was separately, continuously and concurrently added to the reactor over about 3 hours, with continued heating at about 80° to 85°C. The resultant mixture was held for an additional 1 hour at about 80° to 85°C with stirring. Thereafter the so-formed stabile latex composition was cooled for storage and testing.

Latex composition #9 was somewhat similarly prepared by charging into a reactor 81.9 g of deionized water, 2.5 g or triethylamine, 1.7 g of triethylammonium N-cocophthalamate (60.0% solids), 0.4 g of sodium formaldehyde sulfoxylate and 0.4 g of t-butyl hydroperoxide. A pre-emulsion composition comprised of 133.3 g of deionized water, 8.0 g of triethylamine, 13.2 g of triethylammonium N-cocophthalamate, 110.0 g of butyl acrylate, 82.0 g of methylmethacrylate, 28.5g of hydroxyethylmethacrylate and 2.1 g of t-butyl hydroperoxide was prepared and about 26 mL of this pre-emulsion composition was added to the reactor and heated, with stirring, to about 63° to 65°C under a nitrogen blanket. Once the emulsion polymerization reaction began, the remainder of the pre-emulsion composition and, as a separate feed, an initiator solution comprised of 2.1 g of sodium formaldehyde sulfoxylate and 30.0 g of deionized water were separately, continuously and concurrently added to the reactor over a period of about 2 hours, with continued stirring and heating at 63° to 65°C. The resultant polymerization mixture was heated for an additional 1 hours, with continued stirring. Thereafter, the formed stabile latex was cooled to ambient temperature for storage and testing.

Latex composition #10 was prepared from substantially identical ingredients and in a similar manner as that described for composition #9, except that the surfactant utilized was a mix of 50% triethylammonium N-dodecylphthalamate and 50% triethylammonium N-octylphthalamate (60.0% solids).

Latex composition #11 was prepared from substantially identical ingredients and in a similar manner as that described for compositions #5 and #6, except that sodium lauryl sulfate was utilized as a surfactant in place of the ethoxylated sodium lauryl sulfates.

Latex composition #12 was prepared by charging into a suitable set-up reactor 73.5 g of deionized water, 2.0 g of triethylamine, 1.8 g of triethylammonium N-cocophthalamate (60.0% solids), 0.4 g of sodium formaldehyde sulfoxylate and 0.4 g of t-butyl hydroperoxide. About 20 mL of a pre-emulsion composition comprised of 119.8 g of deionized water, 7.0 g of triethylamine, 14.7 g of triethylammonium N-cocophthalamate, 123.2 g of butyl acrylate, 81.2 g of styrene, 10.1 g of methylmethacrylate, 32.2 g of hydroxyethylmethacrylate and 2.4 g of t-butyl hydroperoxide, were added to the reactor and heated to about 63° to 65°C, with stirring under a nitrogen blanket Once the emulsion polymerization reaction began, the remaining pre-emulsion composition and an initiator solution comprised of 2.4 g of sodium formaldehyde sulfoxylate and 35.0 g of deionized water were added, as separate feeds, continuously and concurrently over about 2 hours, with continued stirring and heating at about 63° to 65°C. The reactor charge was heated at this temperature for an additional 1 hour, cooled and discharged for storage and testing.

Latex composition #13 was prepared by charging into a suitably set-up reactor 73.5 g of deionized water 2.0 g of triethylamine and 1.8 g of triethylammonium N-cocophthalamate (60.0% solids) and heating this mixture to about 75°C under an inert gas blanket 0.2 g of $K_2S_2O_8$ was then added to the reactor and, as separate feeds, a pre-emulsion composition comprised of 118.9 g of deionized water, 7.0 g of triethylamine, 14.7 g of triethylammonium N-cocopthalamate (60.0% solids), 123.2g of butyl acrylate, 91.3 g of methylmethacrylate and 32.2 g of hydroxyethylmethacrylate and an initiator solution comprised of 1.0 g of $K_2S_2O_8$ and 30.0 g of deionized water, were continuously and concurrently added to the reactor over about a 2 hour period, with heating at about 75° to 80°C and stirring. This reaction mixture was heated at

the above temperature for an additional 1 hour, cooled to ambient and discharged for storage and testing.

TABLE VII

| LATEX COMPOSITION | CONDITIONS* BAKED | NOT BAKED | SURFACTANT |
|---|---|---|---|
| 1 | 9** | 2 | dimethylethanolammonium N-dodecylphthalamate |
| 2 | 7 | 4 | triethylammonium N-dodecylphthalamate |
| 3 | 8 | 2 | dimethylethanolammonium N-dodecylphthalamate |
| 4 | - | - | triethylammonium N-dodecylphthalamate |
| 5 | 1 | 1 | sodium lauryl sulfate with 20 moles of E.O. |
| 6 | 1 | - | sodium lauryl sulfate with 4 moles of E.O. |
| 7 | 2 | - | sodium lauryl sulfate with 12 moles of E.O. |
| 8 | - | - | triethylammonium N-dodecylphthalamate |
| 9 | 9 | - | triethylammonium N-cocophthalamate |
| 10 | 9 | - | 50/50 mix of triethylammonium N-dodecylphthalamate and triethylammonium N-cocophthalamate |
| 11 | 3 | - | sodium lauryl sulfate |
| 12 | - | - | triethylammonium N-cocophthalamate |
| 13 | - | - | triethylammonium N-cocophthalamate |

TABLE VII CONTINUED

*CONDITIONS Baked = 150°C for 15 minutes and water immersed for at least 3 weeks
Not Baked = dry at ambient for 24 hours and water immersion for at least 3 weeks

** Numerical values assigned on a scale of 1 (worst) to 10 (best), with 10 representing total adhesion and 1 representing all or most of latex film lifted off of glass slide. Values in between indicate various degrees of blistering, lifting, etc.

---

From the foregoing data, it was concluded that latex compositions containing the emulsion polymerization surfactants of the invention consistently outperformed the more traditional latex surfactants (in compositions 5, 6, and 7). It was also concluded that a dramatic difference in water resistance in coating

integrity existed between heat-treated and non-heat treated coatings containing the inventive surfactants. This difference was attributed to the "destructible" nature of the inventive surfactants whereby the N-alkylphthalamate, which exhibits surface active properties in water, when subjected to drying conditions, is chemically converted to N-alkylphthalimide, which is substantially devoid of surface active properties and is substantially water insoluble. It was also noted that dimethylethanolammonium N-dodecylphthalamate, triethylammonium N-cocophthalamate, dimethylethanolammonium N-dodecylphthalamate and a 50/50 mixture of triethylammonium N-dodecylphthalamate and triethylammonium N-cocophthalamate exhibited exceptionally good emulsification properties during latex formation and, coatings containing these particular surfactants exhibited excellent water-insensitivity after drying.

EXAMPLE M

Fabric Softening Characteristics

Sodium N-tallowphthalamate and triethanolammonium octadecylphthalamate were evaluated as fabric softeners and compared against two commercially available fabric softeners (ARQUAD$^R$ 2HT and STEPANTEX$^R$ 6530B) in a conventional domestic laundry machine having typical wash and rinse cycles. White cotton (loop) terry cloth hand towels were prepared for evaluation by CSMA methods DCC-13A and in DCC-13B. The new towels were stripped of mill textile conditioners by washing three times with a standard detergent and 20 g of sodium phosphate, followed by five washings without detergent (all washes were full cycle, hot wash, cold rinse). For each softener tested, as well as for a control (no softener), three towel bundles were added to either the wash cycle or to the rinse cycle. The softeners, at 5 percent solids were dispersed in water and added to the wash or rinse cycle at a use level at about 0.2 percent active softener/weight of towel. After completion of the wash and/or rinse cycle, the towels were dried in a typical domestic dryer for about 50 minutes and evaluated for harshness/softness, with ratings from 1 (harshest) to 3 (softest) and the average results are set forth below:

| SOFTENER | AVERAGE, WASH CYCLE | AVERAGE, RINSE CYCLE |
|---|---|---|
| [1]ARQUAD$^R$ 2HT | 2.5 | 3 |
| [2]STEPANTEX$^R$ 6530B | 2.3 | 2.8 |
| Triethanolammonium N-octadecylphthalamate | 2 | 2.1 |
| sodium N-tallowphthalamate | 2 | 2.2 |
| CONTROL | 1 | 1 |

1. ARQUAD$^R$ 2HT - a registered trademark of AKZO (ARMAK GROUP), Inc. for a dimethylditallow ammonium chloride.
2. STEPANTEX$^R$ 6530B - a registered trademark of Stepan Company for a dialkyl quaternary ammonium methyl sulfate.

As can be seen from the above, the fabric softeners of the invention (alkyl/alkylene phthalamates) exhibited softening characteristics in both wash and rinse cycles, with better results exhibited in the rinse cycle. Further, the fabric softeners of the invention compared favorably with the commercial softeners tested.

EXAMPLE N

Reversible Crude Oil Emulsion/Dispersion/Suspension Characteristics

Sodium N-dodecylphthalamate, sodium N-cocophthalamate, sodium N-isodecyloxypropylphthalamate and sodium N-isododecyloxypropylphthalamate, along with a control (tap water only), were evaluated as emulsifying and/or dispersing and/or suspending agents for a given, heavy (viscous) crude petroleum oil (Dome crude oil, David Field, California) in different water samples, one having a hardness (expressed as $CaCO_3$) of 150 ppm, another having a hardness of 500 ppm, along with a synthetic sea water (NaCl-containing tap water, 150 ppm). In addition, various admixtures of sodium N-dodecylphthalamate and lauryl amine oxide were similarly evaluated with the above petroleum crude oil and synthetic sea water for synergistic emulsification characteristics.

The respective surfactants were prepared as aqueous solutions with the various water samples at a concentration of about 0.1 percent actives. Measured samples (50 mL) of the surfactant solutions were placed in 100 mL graduated cylinders, 2 drops of the crude oil added, the cylinders stoppered and relatively vigorously shaken manually for about 10 to 20 seconds. The degree of emulsification/dispersion, suspension was noted and recorded. The data is set for in Table VIII below.

From the data observed, it was noted that some of the emulsions/dispersions or suspensions did not appear very stabile and sometimes separated within minutes. However, the lauryl amine oxide/sodium N-dodecylphthalamate solutions provided more stabile emulsions which did not separate even after 2 hours. From the data obtained, it was concluded that N-alkylphthalamates emulsified/dispersed crude oil in relatively hard water and that the N-alkyletherphthalamates at least dispersed oil in relatively hard water and in sea water. Additionally, it was concluded that there was a synergistic effect in producing emulsions with a combination of an alkyl amineoxide and N-alkylphthalamtes.

## TABLE VIII

| SURFACTANT | TEST SOLUTIONS: 150 PPM. HRD. WATER pH ABOUT 7 | 500 PPM. HRD. WATER pH ABOUT 7 | SYNTHETIC SEA WATER pH ABOUT 8 |
|---|---|---|---|
| CONTROL | * 0 | 0 | 0 |
| SODIUM N-DODECYLPHTHALAMATE | ++ | d | +/d |
| SODIUM N-COCOPHTHALAMATE | ++ | +/d | +/d/f |
| SODIUM N-ISODECYLOXYPROPYLPHTHALAMATE | d | d | d |
| SODIUM N-ISODODECYLOXYPROPYLPHTHALAMATE | d | d | d |
| [1]AMMONYX[R]-LO AND SODIUM N-DODECYLPHTHALAMATE AT RATIOS OF: | | | |
| 80/20 | - | - | ++ |
| 60/40 | - | - | + |
| 50/50 | - | - | +++ |
| 40/60 | - | - | +++ |
| 30/70 | - | - | +++ |

1. AMMONYX[R] - A registered trademark of Stepan Company for lauryl amine oxide.

+0 - no apparent emulsion/dispersion or suspension
\+ - slight apparent emulsion dispersion
++ - improved apparent emulsion/disperion or suspension
+++ - oil completely emulsified
d - apparent dispersion
d/f - oil apparently dispersed as floc

In order to demonstrate the "reversible" nature of the emulsification properties of the surfactants of the invention, the pH of a sodium N-dodecylphthalamate solution (0.1% actives in 50 mL of 150 ppm hardness water) containing about 2 drops of the aforesaid crude oil was repeatedly adjusted. At relatively high pH values (i.e., above about 7) emulsification of the crude oil readily occurred and at relatively how pH values (i.e., below about 5) de-emulsification of the crude oil readily occurred. A control was prepared from a sample of the 150 ppm hardness water without surfactant but with 2 drops of the above crude oil.

Adjusting the pH of the control from about 13 to about 3 did not produce any emulsions or dispersions. However, when the above N-alkylphthalamate solution was adjusted to a pH of 8.5 the oil readily emulsified. The pH of this emulsion was then dropped by the addition of 1N HCl to a value of about 2.6 and upon agitation, the emulsion broke, almost instantly, with floccing. The pH of this acidified mixture was then raised by addition of 1N NaOH, to a value of about 13.4 and with agitation (about 10-15 seconds of manual shaking) an emulsified solution was noted. The foregoing cycle between relatively high pH and relatively low pH was repeated an additional three times, with essentially identical emulsification/de-emulsification results. During a low (4.9) pH de-emulsification cycle, separated crude oil was removed, by decantation and a pH of the remaining (used) phthalamic solution was adjusted to about 13.5 and fresh crude oil added. Upon agitation, very stabile emulsion was quickly formed. The pH of this emulsion was then dropped to about 7.0 without noticeable de-emulsification. The pH was then further dropped to about 5.2, with a slight flocculation noted. The pH value was then dropped to about 3.5 and the emulsion broke. Upon addition of additional fresh sodium N-dodecylphthalamate, the emulsion re-formed.

From the foregoing data it is concluded that N-alkylphthalamates and N-alkyletherphthalamates are useful in emulsifying viscous petroleum crude oils so as to enable such oils to be transported between select locations. Further, such emulsifications can be readily de-emulsified by adjusting the pH of the emulsion to a value below about pH 5 and the "deactivated" emulsifier reactivated by increasing the pH thereof to a value above about pH 7.

## EXAMPLE O

### Compatibilization/Surfactant Characteristics in Polyurethane/Isocyanurate Systems

Various aromatic polyester polyols are utilized in a variety of polyurethane/isocyanurate foam systems employed in, for example, production of laminated boards, insulating foams, etc. During production of such foam systems, blowing agents such as fluorocarbons, water, carbon dioxide, etc., are utilized and must be blended into the so-called polymer blends. However, blowing agents are typically insoluble or non-dispersable in these blends so that compatibilizing agents or surfactants must be included. For further details see Wood U.S. Patent 4,595,711, (assigned to the instant assignee and which is incorporated herein by reference).

A typical polyol blend was formulated with no surfactant (control) and with three different surfactants of the invention. The two sodium salts (defined below) were solids while the triethylammonium salt was a liquid. The sodium salts were difficult to solubilize in the polyol blend and did not appear to compatibilize the blowing agent into the resin mixture. However, these sodium salts did not appear to interfere with the foam reaction and exhibited fair cell structures, friability, density (reactivity) and overall cell integrity (see Table IX-A, below). On the other hand, the triethylammonium salt solubilized quite well in the polyol mixture and demonstrated good compatibilization or surfactant characteristics with the blowing agent tested (see Table IX-B, below).

TABLE IX-A

| COMPONENT | FORMULATIONS: Ingredients as % by weight | | | |
|---|---|---|---|---|
| | Control | #9 - 1 | #9 - 2 | #9 - 3 |
| [1]POLYOL | 100 | 9.5 | 9.5 | 92.5 |
| PHTHALAMATE | 0.00 | *5.0 | **5.0 | ***7.5 |
| [2]DABCO K-15 | 4.2 | 4.2 | 3.5 | 2.4 |
| [3]POLYCAT - 8 | 1.4 | 1.4 | 1.16 | 0.8 |
| [4]B-8433 | 2.25 | 2.25 | 2.25 | 2.25 |
| [5]R-11 | 47.0 | 47.0 | 40.0 | 23.0 |
| ISOCYANATE | 132.20 | 126.33 | 123.9 | 117.35 |
| CREAM TIME, sec. | 7 | 5 | 6 | 7 |
| GEL TIME, sec. | 11 | 20 | 23 | 26 |
| TACK-FREE TIME, sec. | 20 | 21 | n/a | 28 |
| END OF RISE TIME, sec. | 25 | 50 | 77 | 90 |
| CUP DENSITY (lbs./cubic ft.) | n/a | 1.43 | 1.7 | n/a |
| [6]CELL STRUCTURE | E | F | F | P |
| [6]FRIABILITY | E | F | F | F |
| [6]INTEGRITY | E | P | F | P |

*Sodium N-tallowphthalamate

**Sodium N-dodecylphthalamate

***Triethylammonium N-isododecyloxypropylphthalamate

TABLE IX-A CONTINUED

1. Polyol - prepared substantially in accordance with Example A of Wood, U.S. Patent 4,595,711 except that the hydroxyl number was adjusted to 185 and the final product had a viscosity of about 22,000 centiposes at 25°C.

2. DABCO K-15 - trade name designation of Air Products & Chemicals, Inc., Allentown, PA for potassium octoate.

3. POLYCAT - 8 - trade name designation of Air Products & Chemicals, Inc., Allentown, PA for dimethyl cyclohexylamine.

4. B - 8432 - trade name designation of Goldschmidt Chemical Corp., Hopewell, VA for a polysiloxane surfactant.

5. R-11 - Freon 11, tricholorofluoromethane obtained from E.I. du Pont de Nemours & Company, Wilmington, DE.

6. Visual inspections, E = excellent, G = good, F = fair, P = poor

TABLE IX-B

| Formulation | | |
|---|---|---|
| | Control | 9-4 |
| [1]POLYOL | 100 | 92.5 |
| PHTHALAMATE | 0 | 7.5 |
| [5 7]R-11, max. soluble, g. | 9.10 | 24.7 |

1. ibid.
5. ibid.
7. R - 11 added to polyol in small increments until incompatibility was reached. Endpoint is marked by a slight white/blue haziness, additions beyond this point cause the mixture to become a milky, opaque white.

From the foregoing data it is concluded that the N-alkylphthalamates of the invention function as surfactants in polyurethane/isocyanurate foam systems. Further, it is apparent that the longer chain N-alkylphthalamate exhibited excellent compatibilization characteristics with the blowing agent tested. Yet further, potassium salts of the inventive surfactants are of potential interest since such appear to have a beneficial effect on the catalysis of these foam systems.

## EXAMPLE P

### Industrial Oily Materials Emulsification Characteristics

Certain industrial oily materials, such as linseed oil, tall oil, soybean oil alkyd, linseed oil alkyd, various cuts of distilled petroleum oil, heavy paraffinic oil, asphaltene oil, etc., are useful in a wide variety of applications, such as in road construction, as resin binders, etc., but are extremely difficult to manipulate due to their inherent viscosity, adhesiveness, lack of spreadibility, etc. Attempts to emulsify these oily materials have met with only limited success and typically involved the use of a plurality of high HLB and low HLB emulsifiers and non-aqueous solvents which, at best, form non-stabile emulsions.

For example, linseed oil is used in road constructions as a binding material prior to repairing at least certain roadways. Linseed oil is an extremely sticky and heavy material and is very difficult to apply in a somewhat uniform manner. Heretofore, linseed oil, per se, or in combination with non-aqueous solvents, was loaded onto a specialized truck having a heated tank maintained at a fairly high temperature (i.e, about 90 °

to 100°C). The tank truck included feed pipes leading to spray nodes extended along a manifold to cover a given surface area (i.e., a road lane). The heated linseed oil was pumped through the nozzles, which in some instances were also connected to separate emulsion/solvent solution storage tanks so as to briefly form an emulsion at the nozzle head. This emulsion was then sprayed onto the work surface for manual spreading. Once the linseed oil cooled down and/or the emulsion broke, the linseed oil became very difficult to spread uniformly with manual spreading tools. As will be appreciated, with this type of system, the spray nozzles tend to clog-up and are difficult to clean and workers are exposed to organic vapors and excessive heat.

In accordance with the principles of the invention, linseed oil was readily emulsified into an aqueous system utilizing the phthalamate surfactants (a relatively high HLB emulsifier) in combination with a relatively low HLB emulsifier, such as glycerol stearates, preferably glycerol monostearate, and water. The resulting emulsions were very easy to spray and spread to a desired area. Emulsified linseed oil was stored over extended periods of time without noticeable separation. Further, emulsified linseed oil does not require high temperature for application, thereby providing an economical as well as an environmental advantage.

Linseed Oil Emulsion Formulation

| Component | | Wt. % |
|---|---|---|
| A. | LINSEED OIL | 60.0 |
| | SODIUM N-OCTADECYLPHTHALAMATE | 2.0 |
| | [1]KESSCO$^R$ GLYCERYL MONOSTEARATE PURE | 1.0 |
| B. | WATER | Q.S. 100 |

1. KESSCO$^R$ - Registered trademark of Stepan Company for various organic esters.

Components A, above, were charged into a heatable vessel and heated with agitation to a temperature of about 77° to 80°C. Component B, above, was charged into a separate heatable vessel and also heated to about 77° to 80°C. Thereafter, component A was added to component B with agitation and continued heating for about 30 minutes or until the emulsion formed. Thereafter, agitation was continued and the emulsion was allowed to cool to about 32°C. Agitation was stopped and emulsion transferred for storage and evaluation. Emulsion stability was evaluated at about 43°C for a 1 month period, at room temperature for a 3 month period and at about 2°C for a 1 month period. In each instance, the emulsion was stabile and was easily applied onto glass slides.

Similarly, other industrial oily materials such as polybutylenes, polyethylenes silicones, polysilicones, gums, etc., can readily be emulsified by combining the relatively high HLB phthalamate emulsifiers of the invention with a relatively low HLB emulsifier, up to about 80 percent by weight of an oily material and Q.S. 100 water.

As is apparent from the foregoing specification, the invention is susceptible of being embodied with various alterations and modifications which may differ particularly from those that have been described in the preceding specification and description. For this reason it is to be fully understood that all the foregoing is intended to be merely illustrative. It is not to be construed or interpreted as being restrictive or otherwise limiting of the present invention, excepting as it is set forth and defined in the hereto-appended claims.

## Claims

1. A composition comprising an emulsion of an oil phase and a water phase, wherein the emulsion comprises an emulsifier of the formula:

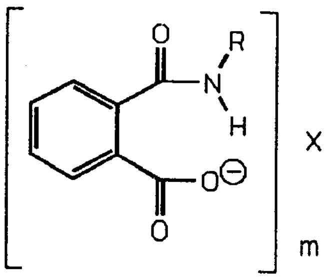

wherein:
R represents $C_1$-$C_{40}$ alkyl, $C_1$-$C_{40}$ alkenyl, alkylaryl where the alkyl portion is $C_1$-$C_{40}$ alkyl, aryl, $C_3$-$C_7$ cycloalkyl, or $R_1$-O-$R_2$ where $R_1$ and $R_2$ independently represent $C_1$-$C_{22}$ alkyl, $C_1$-$C_{22}$ alkenyl, alkylaryl where the alkyl portion is $C_1$-$C_{22}$ alkyl, aryl, or $C_3$-$C_7$ cycloalkyl;
X represents a cation;
m is an integer satisfying the valency of X; and
the composition is at a pH of 5.0 to 10.

2. A composition as claimed in claim 1, wherein R is $C_8$-$C_{40}$ alkyl.

3. A composition as claimed in claim 1 or claim 2, wherein X is a cation selected from the group consisting of sodium, potassium, triethanolammonium, and ammonium.

4. A composition as claimed in any one of the preceding claims, wherein R is $C_{10}$-$C_{22}$ alkyl.

5. A composition as claimed in claim 1, where X is triethanolammonium and R represents $C_{18}$ alkyl.

6. A composition as claimed in claim 1, where X is a sodium ion and R represents $C_{18}$ alkyl.

7. A composition as claimed in claim 1, where X is a sodium ion and R represents $C_{16}$ alkyl.

8. A composition as claimed in any one of the preceding claims, further comprising an ester selected from the group consisting of glyceryl mono-fatty acid esters and polyglyceryl esters.

9. A composition as claimed in any one of the preceding claims, where the composition comprises at least about 0.5% by weight of the emulsifier.

10. A composition as claimed in any one of the preceding claims, wherein the composition is a fast-breaking emulsion having a breakdown time of less than 30 seconds.

11. A composition as claimed in any one of the preceding claims wherein the emulsifier is sodium N-octadecyl phthalamate.

12. A composition as claimed in any one of the preceding claims wherein the weight ratio of the oil phase to the emulsifier is at least about 6.67:1.

13. A composition as claimed in claim 1, where the oil phase comprises mineral oil, isopropyl myristate, aloe vera oil, glycerine, caprylic/capric triglyceride, petrolatum light, avocado oil, sesame seed oil, safflower oil, corn oil, peanut oil, lanolin, acetylated lanolin, melanin, linseed oil, polybutylene, polyethylene, silicone, polysilicone, gum, soybean oil, isopropyl palmitate, octyl isononanoate or mixtures thereof.

European Patent Office

# EUROPEAN SEARCH REPORT

Application Number
EP 95 20 1082

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| E | JOURNAL OF COLLOID AND INTERFACE SCIENCE, vol. 139, no. 2, 15 October 1990 LONDON, pages 381-391, Y. J. UANG ET AL. 'w/o microemulsion studies with mono- and dialkyl amic surfactants' * the whole document * --- | 1-5 | B01F17/00 |
| P,A | RESEARCH DISCLOSURE, no. 310, February 1990 NEW YORK US, pages 158-161, XP 000087057 SAJIC 'conditioning and antidandruff shampoo compositions with amidocarboxy benzoic acids and amidocarboxy benzoates as emulsifiers and suspending agents' * examples * --- | 1-5 | |
| X | DE-A-37 01 719 (BASF) 4 August 1988 * the whole document * --- | 1-4 | |
| A | US-A-2 378 443 (H.G. SMITH ET AL.) * the whole document * --- | 1,2 | TECHNICAL FIELDS SEARCHED (Int.Cl.5) |
| A | US-A-2 101 323 (P. SALZBERG) * the whole document * ----- | 1-5 | B01F |

The present search report has been drawn up for all claims

| Place of search | Date of completion of the search | Examiner |
|---|---|---|
| THE HAGUE | 26 September 1995 | Couckuyt, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document